# EUROPÄISCHE PATENTANMELDUNG

(11) **EP 0 780 394 A1**
(43) Veröffentlichungstag der Anmeldung: **25.06.1997**
(21) Anmeldenummer: 96810863.9
(22) Anmeldetag: 11.12.1996
(51) Int. Cl.: C07D 513/04, A01N 43/90, C07D 333/36

(54) **Mikrobizide**

(30) Priorität: 20.12.1995 CH 3613/95
(71) Anmelder: Novartis AG, 4058 Basel (CH)
(72) Erfinder: Stanetty, Peter, 211 Harmannsdorf (AT); Kunz, Walter, 4104 Oberwil (CH)

(57) **Zusammenfassung**

Die Erfindung betrifft neue mikrobizid und pflanzenimmunisierend wirksame Verbindungen der Formel I, 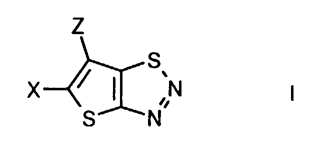 worin bedeuten:
X Wasserstoff, Halogen, C₁-C₄-Alkyl, SR₈, N(R₉)R₁₀, CH₂OH, CHO, COOR₈;
R₈ bis R₁₀ Wasserstoff, C₁-C₄-Alkyl, Benzyl, C₁-C₄-Acyl oder Benzoyl;
Z Wasserstoff, Halogen oder eine Methyl-Gruppe, die unsubstituiert ist oder an die 1-3 gegebenenfalls substituierte Heteroatome O, S und/oder N gebunden sind;
in freier Form oder in Salzform.

Sie können im Pflanzenschutz zur Bekämpfung und Verhütung von Krankheitsbefall verwendet werden.

## Beschreibung

Die Erfindung betrifft neue mikrobizid und pflanzenimmunisierend wirksame Verbindungen der Formel I, 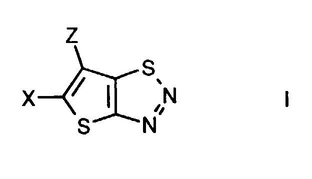 worin bedeuten:
X Wasserstoff, Halogen, C₁-C₄-Alkyl, SR₈, N(R₉)R₁₀, CH₂OH, CHO, COOR₈;
R₈ bis R₁₀ Wasserstoff, C₁-C₄-Alkyl, Benzyl, C₁-C₄-Acyl oder Benzoyl;
Z Wasserstoff, Halogen oder eine Methyl-Gruppe, die unsubstituiert ist oder an die 1-3 gegebenenfalls substituierte Heteroatome O, S und/oder N gebunden sind;
in freier Form oder in Salzform.

Die Erfindung betrifft ferner die Herstellung dieser Verbindungen, agrochemische Mittel, die als Wirkstoff mindestens eine dieser Verbindungen enthalten, sowie die Verwendung der Wirkstoffe oder der Mittel zum Schutz und zur Immunisierung von Pflanzen gegen den Befall durch schädliche Mikroorganismen, beispielsweise Pilze, Bakterien und Viren.

Die Verbindungen der Formel I und gegebenenfalls ihre Tautomeren können als Salze vorliegen. Verbindungen der Formel 1, die mindestens ein basisches Zentrum aufweisen, können Säureadditionssalze bilden. Diese werden beispielsweise mit Mineralsäuren, z.B. Schwefelsäure, einer Phosphorsäure oder einer Halogenwasserstoffsäure, mit organischen Carbonsäuren, wie z.B. Essigsäure, Oxal-, Malon-, Malein-, Fumar- oder Phthalsäure, mit Hydroxycarbonsäuren, z.B. Ascorbin-, Milch-, Äpfel-, Wein- oder Zitronensäure, oder mit Benzoesäure, oder mit organischen Sulfonsäuren, wie z.B. Methan- oder p-Toluolsulfonsäure, gebildet.
Femer können Verbindungen der Formel I mit mindestens einer aciden Gruppe Salze mit Basen bilden. Geeignete Salze mit Basen sind beispielsweise Metallsalze, wie Alkali- oder Erdalkalimetallsalze, z.B. Natrium-, Kalium- oder Magnesiumsalze, oder Salze mit Ammoniak oder einem organischen Amin, wie Morpholin, Piperidin, Pyrrolidin, einem Mono-, Di- oder Triniederalkylamin, z. B. Ethyl-, Diethyl-, Triethyl- oder Dimethyl-propylamin, oder einem Mono-, Di- oder Trihydroxyniederalkylamin, z.B. Mono-, Di- oder Triethanolamin. Weiterhin können gegebenenfalls entsprechende innere Salze gebildet werden. Bevorzugt sind im Rahmen der Erfindung agrochemisch vorteilhafte Salze.

Die vor- und nachstehend verwendeten Allgemeinbegriffe haben, sofem nicht abweichend definiert, die nachfolgend aufgeführten Bedeutungen:
Kohlenwasserstoffreste können gesättigt oder ungesättigt, offenkettig oder cyclisch, oder gemischt offenkettig und cyclisch, wie z.B. Cyclopropylmethyl oder Benzyl, sein.

Alkylgruppen sind, je nach Anzahl der Kohlenstoffatome, geradkettig oder verzweigt und stehen beispielsweise für Methyl, Ethyl, n-Propyl, iso-Propyl, n-Butyl, sek.-Butyl, iso-Butyl, tert.-Butyl, sek. Amyl, tert. Amyl, 1-Hexyl oder 3-Hexyl.

Ungesättigte Kohlenwasserstoffreste sind Alkenyl-, Alkinyl- oder Alkeninylgruppen mit höchstens 3 Mehrfachbindungen, wie z.B. Butadienyl, Hexatrienyl, 2-Penten-4-inyl.

Unter Alkenyl ist geradkettiges oder verzweigtes Alkenyl zu verstehen, wie z.B. Allyl, Methallyl, 1-Methylvinyl oder But-2-en-1-yl. Bevorzugt sind Alkenylreste mit einer Kettenlänge von 3 bis4 Kohlenstoffatomen.

Alkinyl kann ebenfalls, je nach Anzahl der Kohlenstoffatome, geradkettig oder verzweigt sein, wie z.B. Propargyl, But-1-in-1-yl, But-1-in-3-yl. Bevorzugt ist Propargyl.

Cyclische ungesättigte Kohlenwasserstoffreste können aromatisch sein wie z.B. Phenyl und Naphthyl, oder nicht aromatisch wie z.B. Cyclopentenyl, Cyclohexenyl, Cycloheptenyl und Cyclooctadienyl, oder teilaromatisch wie z.B. Tetrahydronaphthyl und Indanyl.

Halogen bzw. Halo stehen für Fluor, Chlor, Brom oder Jod, vorzugsweise für Fluor, Chlor oder Brom.

Haloalkyl kann gleiche oder verschiedene Halogenatome enthalten, z.B. Fluormethyl, Difluormethyl, Difluorchlormethyl, Trifluormethyl, Chlormethyl, Dichlormethyl, Trichlormethyl, 2,2,2-Trifluorethyl, 2-Fluorethyl, 2-Chlorethyl, 2,2,2-Trichlorethyl, 3,3,3-Trifluorpropyl.

Alkoxy ist beispielsweise Methoxy, Ethoxy, Propyloxy, i-Propyloxy, n-Butyloxy, iso-Butyloxy, sek.-Butyloxy und tert.-Butyloxy; vorzugsweise Methoxy und Ethoxy.

Haloalkoxy ist z.B. Difluormethoxy, Trifluormethoxy, 2,2,2-Trifluorethoxy, 1,1,2,2-Tetrafluorethoxy, 2-Fluorethoxy, 2-Chlorethoxy und 2,2-Difluorethoxy.

Cycloalkyl ist Cyclopropyl, Cyclobutyl, Cyclopentyl oder Cyclohexyl.

Alkanoyl ist entweder geradkettig oder verzweigt, Beispiele sind Formyl, Acetyl, Propionyl, Butyryl, Pivaloyl oder Octanoyl.

Unter einem Heterocyclylrest werden 5- oder 6-gliedrige, aromatische und nicht aromatische Ringe mit Heteroatomen N, O und/oder S verstanden. Weiterhin kann einem solchen an das Restmolekül gebundenen Heterocyclylrest eine unsubstituierte oder substituierte Benzo-Gruppe ankondensiert sein. Beispiele für Heterocyclylgruppen sind Pyridyl, Pyrimidinyl, Imidazolyl, Thiazolyl, 1,3,4-Thiadiazolyl, Triazolyl, Thienyl, Furanyl, Pyrrolyl, Morpholinyl, Oxazolyl und die entsprechenden partiell oder total hydrierten Ringe. Beispiele für Heterocyclylgruppen mit ankondensierter Benzogruppe sind Chinolyl, Isochinolyl, Benzoxazolyl, Chinoxalinyl, Benzothiazolyl, Benzimidazolyl, Indolyl, Indolinyl.

Alle vorgenannten Aufzählungen sind beispielhaft und stellen keine Limitierungen dar.

Bevorzugte Gruppen sind:
(1) Verbindungen der Formel I, worin bedeuten:
   Z Wasserstoff, CN, CO-A, CS-A, CH(OR₁)_{2,}
   und worin ferner die übrigen Substituenten folgende Bedeutungen haben:
   A Wasserstoff, Halogen, OR₂, SR₂, N(R₃)R₄, NH-OR₅, O-N(=C)ₙ(R₆)R₇ oder NH-N(=C)ₙ(R₆)R₇;
   R₁ gleich oder verschieden C₁-C₄-Alkyl, das unsubstituiert oder durch Phenyl, C₁-C₂ Alkoxy, Phenoxy oder Benzyloxy substituiert ist; oder
   zwei OR₁ zusammen mit dem Kohlenstoffatom, an das sie gebunden sind, eine cyclische Acetalgruppe, die unsubstituiert oder mit C₁-C₃-Alkyl, Phenyl, Benzyl, Hydroxy, oder C₁-C₃-Hydroxyalkyl substituiert ist;
   R₂ bis R₇ Wasserstoff, ein gegebenenfalls substituierter, bis zu 8 Kohlenstoffatome enthaltender offenkettiger, gesättigter oder ungesättigter Kohlenwasserstoffrest, ein gegebenenfalls substituierter, bis zu 10 Kohlenstoffatome enthaltender cyclischer, gesättigter oder ungesättigter Kohlenwasserstoffrest, gegebenenfalls substituiertes Benzyl oder Phenethyl, eine gegebenenfalls substituierte, bis zu 8 Kohlenstoffatome enthaltende Acylgruppe, eine gegebenenfalls substituierte Benzoylgruppe oder ein gegebenenfalls substituierter Heterocyclylrest; oder
   R₃ und R₄ zusammen mit dem Stickstoffatom, an das sie gebunden sind, ein 5- oder 6- gliedriger, gegebenenfalls substituierter Heterocyclus mit 1-3 Heteroatomen O, S, und/oder N;
   R₆ und R₇ zusammen mit dem Atom, an welches sie gebunden sind, ein 5- bis 7-gliedriger, gegebenenfalls substituierter carbocyclischer oder heterocyclischer Ring mit 1-3 Heteroatomen O, S, und/oder N;
   n 0 oder 1; und worin
   X die für Formel I angegebenen Bedeutungen hat.
(2) Verbindungen, worin bedeuten:
   R₁ C₁-C₄-Alkyl, oder zwei OR₁ zusammen mit dem Kohlenstoffatom, an das sie gebunden sind, eine 5-7 gliedrige, cyclische Acetalgruppe, die unsubstituiert oder mit C₁-C₃-Alkyl substituiert ist;
   R₂ und R₃ Wasserstoff, C₁-C₈-Alkyl, das unsubstituiert oder mit 1-5 Halogenatomen, C₃-C₆-Cycloalkyl, C₁-C₄-Alkoxy, Phenoxy, Benzyloxy C₁-C₄-Acyloxy, Benzoyloxy, Hydroxy, Nitro, Cyano, C₁-C₄-Alkanoyl, Benzoyl, Carboxyl, C₁-C₄-Alkoxycarbonyl, Benzyloxycarbonyl, Amino, C₁-C₄-Alkylamino, C₁-C₄-Dialkylamino oder Heterocyclyl substituiert ist, C₃-C₆-Alkenyl, das unsubstituiert oder mit 1-5 Halogenatomen substituiert ist, C₃-C₆-Alkinyl, C₃-C₆-Cycloalkyl, C₁-C₄-Alkanoyl, Phenyl, Benzyl oder Phenethyl, deren Phenylringe unsubstituiert oder ein bis dreifach durch Halogen, Hydroxy, C₁-C₄-Alkyl, Halogen-C₁-C₂-alkyl, C₁-C₂-Alkoxy, Halogen-C₁-C₂-alkoxy oder Nitro substituiert sind, Naphthyl, Benzoyl, oder Heterocyclyl, die unsubstituiert oder ein- bis dreifach gleich oder verschieden durch Halogen, C₁-C₂-Alkyl, Halogenmethyl oder Nitro substituiert sind;
   R₄ Wasserstoff, C₁-C₆-Alkyl, Benzyl; oder
   R₃ und R₄ zusammen mit dem Stickstoffatom, an welches sie gebunden sind, ein 5- oder 6- gliedriger Ring mit 1-2 Heteroatomen O, S, und/oder N,, wobei die genannten Ringe unsubstituiert oder ein- bis zweifach gleich oder verschieden durch Halogen, C₁-C₃-Alkyl oder C₁-C₂-Alkoxycarbonyl substituiert sind;
   R₅, R₆, R₇, unabhängig voneinander Wasserstoff, C₁-C₆-Alkyl, C₃-C₄-Alkenyl, C₃-C₄-Alkinyl, C₃-C₆-Cycloaykyo, Phenyl oder Benzyl, deren Phenylringe unsubstituiert oder ein bis zweifach durch Halogen, Hydroxy, C₁-C₂-Alkyl, C₁-C₂-Alkoxy, Halogenmethyl oder Halogenmethoxy substituiert sind; oder
   R₆ und R₇ zusammen mit dem Atom, an welches sie gebunden sind, ein 5- bis 7-gliedriger carbocyclischer oder heterocyclischer Ring mit 1-3 Heteroatomen O, S und /oder N, wobei die genannten Ringe unsubstituiert oder ein- bis dreifach gleich oder verschieden durch Halogen, oder C₁-C₃-Alkyl substituiert sind; und worin
   X die für Formel I angegebenen Bedeutungen hat.
(3) Verbindungen der Formel I, worin bedeuten:
   Z CO-A, CS-A, CH(OR₁)_{2.}, und worin
   X die für Formel I angegebenen Bedeutungen hat.
(4) Verbindungen der Formel I, worin bedeuten:
   Z CO-A;
   A Wasserstoff, OR₂, SR₂, N(R₃)R₄, NH-OR₅, O-N(=C)ₙ(R₆)R₇ oder NH-N(=C)ₙ(R₆)R₇. R₂ und R₃ Wasserstoff, C₁-C₈-Alkyl, das unsubstituiert oder mit 1-5 Halogenatomen, C₃-C₆-Cycloalkyl, C₁-C₄-Alkoxy, Phenoxy, Benzyloxy, C₁-C₄-Acyloxy, Benzoyloxy, Hydroxy, Nitro, Cyano, C₁-C₄-Alkanoyl, Benzoyl, Carboxyl, C₁-C₄-Alkoxycarbonyl, Benzyloxycarbonyl, Amino, C₁-C₄-Alkylamino, C₁-C₄-Dialkylamino oder Heterocyclyl substituiert ist, C₃-C₆-Alkenyl, das unsubstituiert oder mit 1-5 Halogenatomen substituiert ist, C₃-C₆-Alkinyl, C₃-C₆-Cycloalkyl, C₁-C₄-Alkanoyl, Phenyl, Benzyl oder Phenethyl, deren Phenylringe unsubstituiert oder ein bis dreifach durch Halogen, Hydroxy, C₁-C₄-Alkyl, Halogen-C₁-C₂-alkyl, C₁-C₂-Alkoxy, Halogen-C₁-C₂-alkoxy oder Nitro substituiert sind, Naphthyl, Benzoyl, oder Heterocyclyl, die unsubstituiert oder ein- bis dreifach gleich oder verschieden durch Halogen, C₁-C₂-Alkyl, Halogenmethyl oder Nitro substituiert sind, oder
   R₃ und R₄ zusammen mit dem Stickstoffatom, an welches sie gebunden sind, ein 5- oder 6- gliedriger Ring mit 1-2 Heteroatomen O, S, und/oder N, wobei die genannten Ringe unsubstituiert oder ein- bis zweifach gleich oder verschieden durch Halogen oder C₁-C₃-Alkyl substituiert sind;
   R₅, R₆, R₇, unabhängig voneinander Wasserstoff, C₁-C₆-Alkyl, C₃-C₄-Alkenyl, C₃-C₄-Alkinyl, C₃-C₆-Cycloalkyl, Phenyl oder Benzyl, deren Phenylringe unsubstituiert oder ein bis zweifach durch Halogen, Hydroxy, C₁-C₂-Alkyl, C₁-C₂-Alkoxy, Halogenmethyl oder Halogenmethoxy substituiert sind; oder
   R₆ und R₇ zusammen mit dem Atom, an welches sie gebunden sind, ein 5- bis 7-gliedriger carbocyclischer oder heterocyclischer Ring mit 1-3 Heteroatomen O, S und/oder N, wobei die genannten Ringe unsubstituiert oder ein- bis dreifach gleich oder verschieden durch Halogen, C₁-C₃-Alkyl oder C₁-C₂-Alkoxycarbonyl substituiert sind; und worin
   X die für Formel I angegebenen Bedeutungen hat.
(5) Verbindungen der Formel I, worin bedeuten:
   Z CO-A;
   A OR₂ oder SR₂ davon insbesondere solche, worin bedeuten:
   R₂ Wasserstoff, C₁-C₆-Alkyl, das unsubstituiert oder mit 1-3 Halogenatomen, C₃-C₆-Cycloalkyl, C₁-C₂-Alkoxy, Phenoxy, Hydroxy, Cyano, C₁-C₂-Alkanoyl, Benzoyl, Carboxyl, C₁-C₄-Alkoxycarbonyl, Benzyloxycarbonyl, C₁-C₂-Acyloxy, Benzoyloxy, Amino, C₁-C₄-Alkylamino oder C₁-C₄-Dialkylamino substituiert ist, C₃-C₄-Alkenyl, das unsubstituiert oder mit 1-3 Halogenatomen substituiert ist, C₃-C₄-Alkinyl, C₃-C₆-Cycloalkyl, Phenyl, Benzyl oder Phenethyl, deren Phenylringe unsubstituiert oder ein bis zweifach durch Halogen, Hydroxy, C₁-C₄-Alkyl, Halogen-C₁-C₂-alkyl, C₁-C₂-Alkoxy, Halogen-C₁-C₂-alkoxy oder Nitro substituiert sind;
(6) Verbindungen der Formel I, worin
   Z CO-OR₂ bedeutet.
(7) Verbindungen der Formel I, worin bedeuten:
   Z CN oder CO-N(R₃)R₄ ;
   R₃ Wasserstoff, C₁-C₅-Alkyl, das unsubstituiert oder mit 1-3 Halogenatomen, C₃-C₆-Cycloalkyl, C₁-C₂-Alkoxy, Phenoxy, Hydroxy, Cyano, C₁-C₂-Alkanoyl, Benzoyl, Carboxyl, C₁-C₄-Alkoxycarbonyl, Benzyloxycarbonyl, C₁-C₂-Acyloxy, Benzoyloxy, Amino, C₁-C₄-Alkylamino oder C₁-C₄-Dialkylamino substituiert ist, C₃-C₄-Alkenyl, das unsubstituiert oder mit 1-3 Halogenatomen substituiert ist, C₃-C₄-Alkinyl, C₃-C₆-Cycloalkyl, Phenyl, Benzyl oder Phenethyl, deren Phenylringe unsubstituiert oder ein bis zweifach durch Halogen, Hydroxy, C₁-C₄-Alkyl, Halogen-C₁-C₂-alkyl, C₁-C₂-Alkoxy, Halogen-C₁-C₂-alkoxy oder Nitro substituiert sind;
   R₄ Wasserstoff, C₁-C₆-Alkyl, Benzyl; oder
   R₃ und R₄ zusammen mit dem Stickstoffatom, an das sie gebunden sind, Pyrrolidin, Piperidin, Morpholin oder Dimethylmorpholin; und worin
   X die für Formel I angegebenen Bedeutungen hat.
(8) Verbindungen der Formel 1, worin bedeuten
   Z CHO oder CH(OR₁)₂;
   R₁ gleich oder verschieden C₁-C₄-Alkyl, das unsubstituiert oder durch Phenyl, C₁-C₂ Alkoxy, Phenoxy oder Benzyloxy substituiert sein kann; oder zwei OR₁ zusammen mit dem Kohlenstoffatom, an das sie gebunden sind eine 5 oder 6-gliedrige, cyclische Acetalgruppe, die unsubstituiert oder mit C₁-C₃-Alkyl, Phenyl, Benzyl, Hydroxy, oder C₁-C₃-Hydroxyalkyl substituiert ist; und worin
   X die für Formel I angegebenen Bedeutungen hat.
(9) Verbindungen der Formel I, worin
   Z Wasserstoff, Methyl, Halogen, C₁-C₄-Alkoxymethyl oder Halogenmethyl bedeuten.
(10) Verbindungen der Formel I, worin bedeuten:
   X Wasserstoff, Halogen, Methyl, Mercapto, Methylthio oder Amino;
   insbesondere Wasserstoff.

Verbindungen der Formel I können gemäss Reaktionsschema 1 wie folgt hergestellt werden: 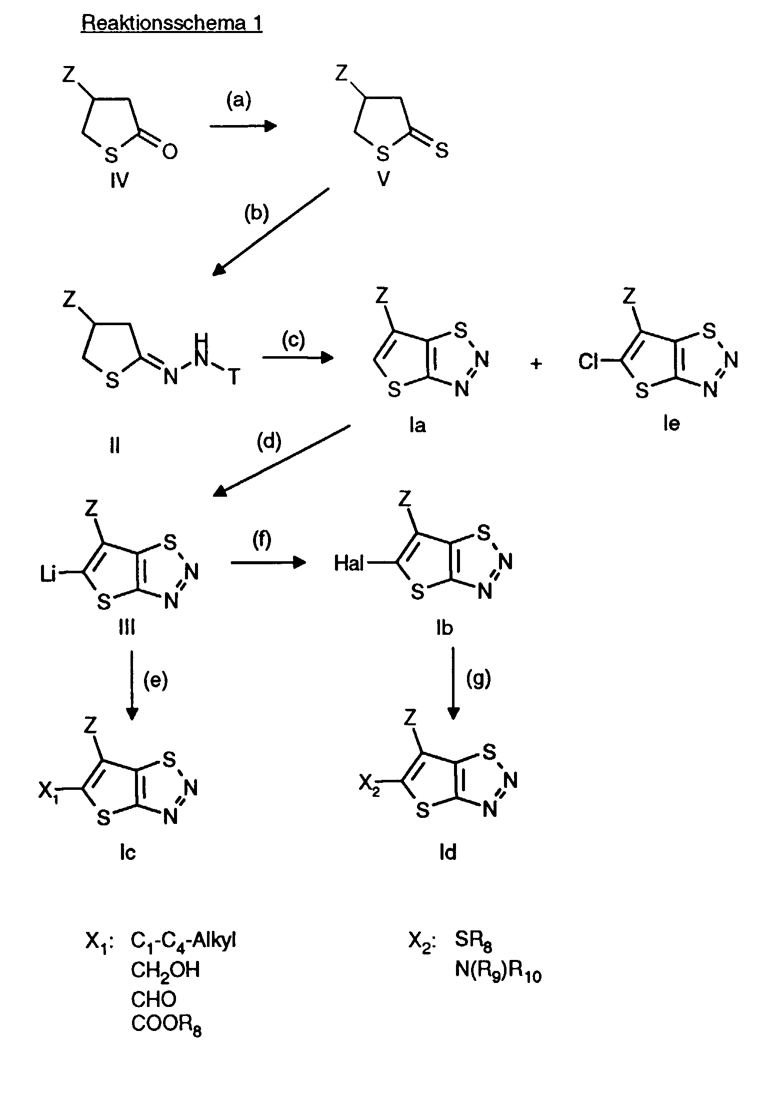
(a) gewünschtenfalls Thionierung einer Verbindung der Formel IV mit 4-Methoxyphenylthiophosphonsäure-cyclodithioanhydrid ("Lawesson-Reagens") oder Phosphorpentasulfid in einem inerten Lösungsmittel wie z.B. Toluol oder Xylol zu einem Dithiolacton der Formel V.
(b) Umsetzung einer Verbindung der Formel V mit einem Hydrazin-Derivat der Formel H₂N-NH-T, worin T eine Gruppe CO-Y, CONY₂ oder SO₂-Y und Y Wasserstoff, gegebenenfalls substituiertes C₁-C₈₋-Alkyl oder gegebenenfalls substituiertes Aryl bedeuten oder T eine Gruppe COO-U und U ein gegebenenfalls substituiertes C₁-C₈₋-Alkyl oder gegebenenfalls substituiertes Aryl bedeutet, in einem polaren Lösungsmittel, z.B. einem Alkohol wie B. Ethanol oder Methanol, zu einer Verbindung der Formel II.
(c) Cyclisierung einer Verbindung der Formel II mit Thionylchlorid nach Hurd-Mori (C. D. Hurd, R. J. Mori, J. *Am. Chem. Soc.* 77, 5359, (1955) zu einem Thieno-1,2,3-thiadiazol der Formel la. Dabei entsteht ebenfalls die chlorierte Verbindung le, je nach Reaktionsbedingungen bis zu 25%.
(d) Umsetzung von geeigneten la mit einer lithiumorganischen Verbindung wie z.B.Butyllithium, sec.Butyllithium, Hexyllithium, Lithiumdiisopropylamid (LDA), Lithiumhexamethyldisilazid oder Lithiumtetramethylpiperidid (LTMP) in einem aprotischen Lösungsmittel wie z.B. THF, Diethylether oder Hexan in An- oder Abwesenheit eines zusätzlichen Komplexierungsmittels bei -100°C bis +20°C zu einer lithiumorganischen Verbindung der Formel III.
(e) Umsetzung von III mit einem C₁-C₄-Alkylhalogenid bzw. Formaldehyd bzw. Dimethylformamid bzw. CO₂ und gegebenenfalls anschliessender Veresterung;
(f) Umsetzung von III mit Halogen, bzw. mit Hexachlorethan.
(g) Umsetzung von Ib mit HSR₈ oder HN(R₉)R₄ unter basischen Bedingungen.
   Verbindungen der Formel IV, worin Z Wasserstoff bedeutet, sind bekannt.
   Verbindungen der Formel IV, worin Z die für Formel I genannten Bedeutungen hat, aber nicht Wasserstoff ist, können gemäss Reaktionsschema 2 wie folgt hergestellt werden: 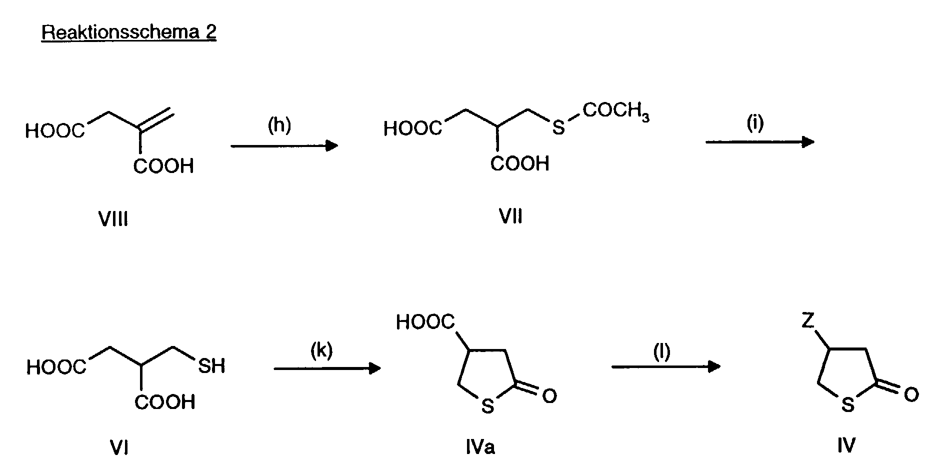
(h) Umsetzung von Itaconsäure der Formel VIII mit Thioessigsäure zu Acetylthiomethylbutandisäure der Formel VII (P. R. Dennis et.al. Biochemistry, 25, 1605, (1986); Beil. Vol. 3, E III, S. 933).
(i) Hydrolyse von Acetylthiomethylbutandisäure der Formel VII zu Mercaptomethylbutandisäure der Formel VI (J. P. Danehy, Int. J. Sulfur Chem. Part C, 6, 159, (1971); Beil. Vol. 3, E III, S. 933, Vol. 3. E IV, S. 1155).
(k) Cyclisierung von Mercaptomethylbutandisäure durch Erhitzen auf 100-160°C unter Abspaltung von Wasser zu Tetrahydro-5-oxo-3-thiophencarbonsäure der Formel V (US 90-583537; Beil. Vol. 18, E III/IV, S. 5265).
(l) Umwandlung der Carboxylgruppe in eine Gruppe Z, worin Z die für Formel I genannten Bedeutungen hat, nach bekannten Methoden, analog Reaktionsschema 8.
   Ferner können Verbindungen der Formel I und If bis Ik gemäss den Reaktionsschemata 4-8, wie folgt hergestellt werden: 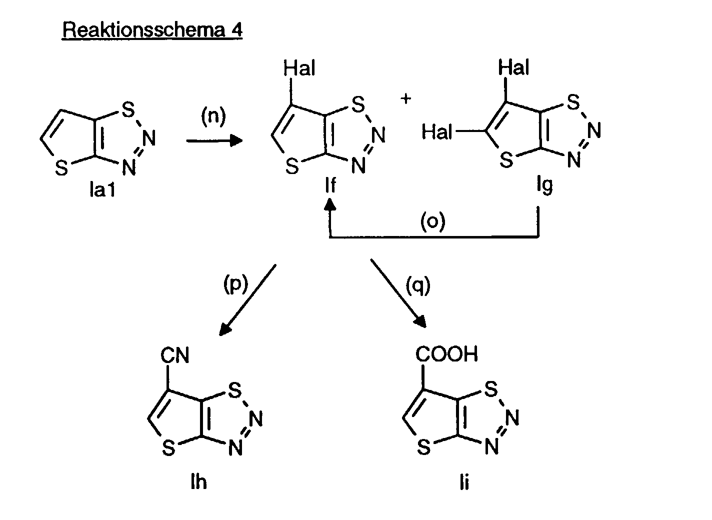
(n) Halogenierung mit einem Halogen mit oder ohne Katalysator (wie Fe(Hal)₃, Sn(Hal)₂) Synth.Comm. 11, 25 (1981); J.Het.Chem. 32,791; J.Chem.Soc. (8)1971,79; Halogenierung mit PhSeHal ( Hal = Cl, Br) in Gegenwart von Al(Hal)₃ ( Phosphorous, Sulfur and Silicon 53, 29 (1990)), Halogenierung mit Sulfurylhalogenid oder N-Halosuccinimid ( Houben-Weyl, Heteroarene I, Teil 1 Band E 6a S. 415 ff) Halogenierung in Gegenwart eines Puffers ( J.Het.Chem. 11,205 (1974).
(o) Reduktion z.B. mit Zink/H⁺ (Bull.Chem.Soc.Jp. 66,2033); mit Na₂Te resp. Te/NaOH (Angew. Chem. 1967,1106); durch Behandlung mit Alkyllithium und anschliesender Protonierung ( Houben Weyl s. oben S.411 ff); Enthalogenierung mittels Cu/Chinolin.
(p) Reaktion mit CuCN in Gegenwart eines Komplexbildners wie Pyridin, Methylpyridin oder Chinolin, in einem aprotischen, polaren Lösungsmittel (Rosenmund-vonBraun-Reaktion).
(q) Reaktion mit Kohlenmonoxid in Gegenwart eines Pd-Katalysators (Heck-Reaktion). 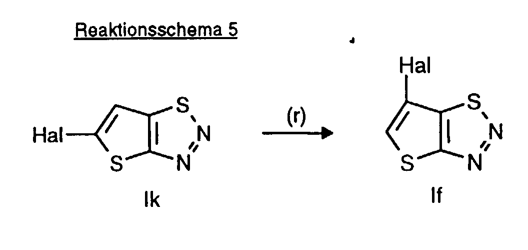
(r ) Umlagerung mittels Zeolith ( Angew. Chem. 26, 470 (1987)); mittels Alkaliamid (Na/NH₃) Rec.trav.Chim. 93,33 (1974)); Synth.Comm. 20,1697 (1990)); J.Org.Chem 36,2690 (1971)).
   Die in Schemata 4 und 5 skizzierten Umsetzungen können teilweise Houben Weyl, Heteroarene I, Teil 1, Band 6a oder "Thiophene and its Derivatives", Heterocyclic Compounds, Edit. Weissberger/Taylor, New York 1992, entnommen werden. 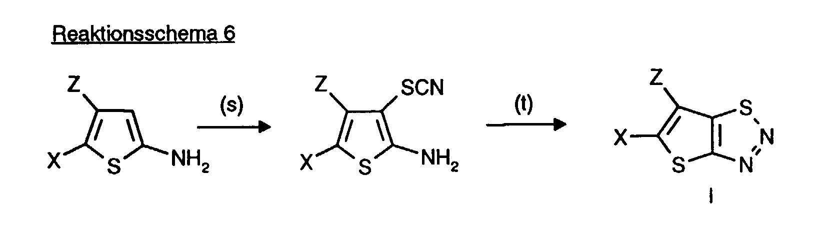
(s) Rhodanierung mit einem Rhodanid MSCN (M=Alkali- oder NH₄⊕ oder Schwermetallatom das mehrwertig sein kann (z.B.Pb(SCN)₂) in Gegenwart eines Oxidationsmittels wie H₂O₂ in inertem Lösungsmittel bei -20°C bis 100°C (bevorzugt -10° bis +50°C).
(t) Diazotierung oder vorgängige Reduktion der -SCN-Gruppe zu -SH z.B. mittels NaBH₄ in inertem Lösungmittel (z.B. Alkohol, H₂O) bei -10° bis 80°C (bevorzugt 0° bis 60°) und anschliessende Diazotierung mit Alkali- oder Alkylnitrit in inertem Lösungsmittel ggf. in Gegenwart einer starken Säure (H₂SO₄, HCl) bei -30°C bis + 50°C (bevorzugt -20°C bis +30°C). 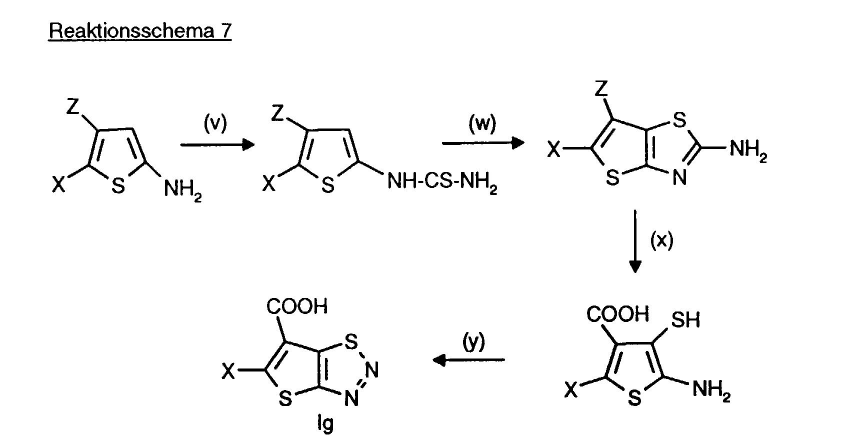
(v) SCN-/Säure, z.B. H₂SO₄/inertes Lösungsmittel
(w) Oxidationsmittel z.B. SO₂Cl₂ oder Br₂
(x) Starke wässrige Base, z.B. Kalilauge,
   Die Reaktionen (v), (w) und (x) sind z.B. analog Org. Synthesis Coll. Vol. III, S. 76; oder J. Het. Chem. 17, 1325 (1980) oder US-5374 737 durchführbar.
(y) Diazotierung mit Alkali- oder Alkylnitrit in inertem Lösungsmittel ggf. in Gegenwart einer starken Säure (H₂SO₄ HCl) bei -30°C bis + 50°C (bevorzugt -20°C bis +30°C).

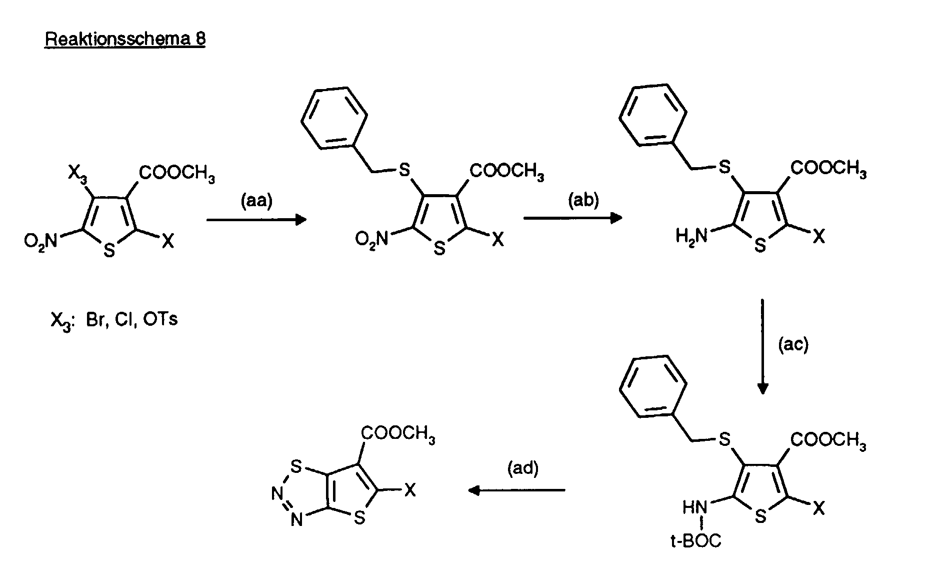

(aa) Substitution einer Abgangsgruppe X3 mit Benzylmercaptan in Gegenwart einer Base wie z.B. K₂O₃, Pyridin oder Triethylamin
(ab) Reduktion mit Eisen in Essigsäure
(ac) Umsetzung mit (t-BOC)₂O
(ad) Diazotierung mit Alkali- oder Alkylnitrit in einem Gemisch aus Essigsäure und Salzsäure

Verbindungen der Formel I, worin Z COOH bedeutet, können nach bekannten Methoden in andere Verbindungen der allgemeinen Formel I, worin Z die für Formel I genannten Bedeutungen hat, umgewandelt werden. So können z.B. die Carbonsäuren mit Thionylchlorid in die entsprechenden Carbonsäurechloride übergeführt werden, aus denen sich die Gruppe Z gemäss Reaktionsschema 9 wie folgt umwandeln lässt: 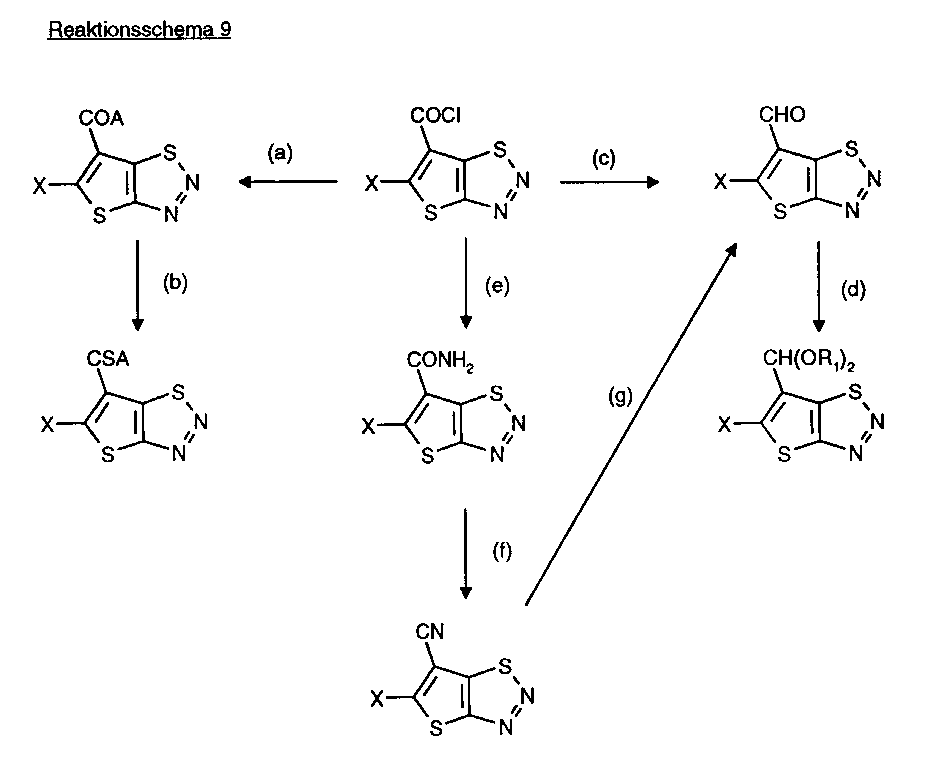
(a) Umsetzung mit M-A, worin M Wasserstoff, Li⁺, Na⁺, K⁺, 1/2Mg²⁺ oder ein quaternäres Ammoniumion und A die für Formel I angegebenen Bedeutungen hat
(b) Umsetzung mit einem Thionierungsmittel, z.B. Phosphorpentasulfid oder 4-Methoxyphenylthiophosphonsäure-cyclodithioanhydrid ("Lawesson-Reagens");
(c) Reduktion z.B. mit Wasserstoff/Katalysator oder Natriumborhydrid;
(d) Säurekatalysierte Umsetzung mit dem entsprechenden Alkohol oder Diol unter Entfernung von Wasser
(e) Umsetzung mit NH₃
(f) Reaktion mit einem Wasserabspaltungsmittel, z.B. SOCl₂; POCl₃ oder COCl₂
(g) Reduktion mit Diisobutylaluminiumhydrid

Verbindungen der Formel I, worin X = COOH können durch Decarboxylierung zu X = H oder durch Halodecarboxylierung zu X = Halogen umgewandelt werden.

Geeignete Basen sind z. B. Alkalimetall- oder Erdalkalimetall-hydroxide, -hydride, -amide,-alkanolate, -carbonate, -dialkylamide oder -alkylsilylamide, Alkylamine, Alkylendiamine, gegebenenfalls N-alkylierte, gegebenenfalls ungesättigte Cycloalkylamine, basische Heterocyclen, Ammoniumhydroxide sowie carbocyclische Amine. Beispielhaft seien Natrium-hydroxid, -hydrid, -amid, -methanolat, -carbonat, Kalium-tert.-butanolat, -carbonat, Lithiumdiisopropylamid, Kalium-bis(trimethylsilyl)-amid, Calciumhydrid, Triethylamin, Triethylendiamin, Cyclohexylamin, N-Cyclohexyl-N,N-dimethyl-amin, N,N-Diethylanilin, Pyridin, 4-(N,N-Dimethylamino)pyridin, N-Methylmorpholin, Benzyl-trimethyl-ammoniumhydroxid sowie 1,8-Diazabicyclo[5.4.0]undec-5-en (DBU) genannt.

Abgangsgruppen sind z. B. Chlor, Brom, lod, C₁-C₈-Alkylthio, wie Methylthio, Ethylthio oder Propylthio, C₁-C₈-Alkanoyloxy, wie Acetoxy, (Halogen-)C₁-C₈- Alkansulfonyloxy, wie Methansulfonyloxy, Ethansulfonyloxy oder Trifluormethansulfonyloxy, oder gegebenenfalls substituiertes Phenylsulfonyloxy, wie Benzolsulfonyloxy oder p-Toluolsulfonyloxy, Imidazolyl, Hydroxy oder Wasser, bevorzugt Chlor, Brom, lod. Trifluormethansulfonyloxy und p-Toluolsulfonyloxy.

Die Reaktionspartner können als solche, d. h. ohne Zusatz eines Lösungs- oder Verdünnungsmittels, z. B. in der Schmelze, miteinander umgesetzt werden. Zumeist ist jedoch der Zusatz eines inerten Lösungs- oder Verdünnungsmittels oder eines Gemisches derselben vorteilhaft. Als Beispiele für solche Lösungs- oder Verdünnungsmittel seien genannt: aromatische, aliphatische und alicyclische Kohlenwasserstoffe und Halogenkohlenwasserstoffe, wie Benzol, Toluol, Xylol, Chlorbenzol, Brombenzol, Petrolether, Hexan, Cyclohexan, Dichlormethan, Trichlormethan, Dichlorethan oder Trichlorethan; Ether, wie Diethylether, tert.-Butylmethylether, Tetrahydrofuran oder Dioxan; Ketone, wie Aceton oder Methylethylketon; Alkohole, wie Methanol, Ethanol, Propanol, Butanol, Ethylenglycol oder Glycerin; Ester, wie Essigsäureethylester oder Essigsäurebutylester; Amide, wie N,N-Dimethylformamid, N,N-Dimethylacetamid, N-Methylpyrrolidon oder Hexamethylphosphorsäuretriamid; Nitrile, wie Acetonitril; und Sulfoxide, wie Dimethylsulfoxid. Auch im Ueberschuss eingesetzte Basen, wie Triethylamin, Pyridin, N-Methylmorpholin oder N,N-Diäthylanilin, können als Lösungs- oder Verdünnungsmittel dienen.

Die Umsetzung kann auch unter Phasentransferkatalyse in einem organischen Lösungsmittel, z.B. Methylenchlorid oder Toluol, in Gegenwart einer wässrigen basischen Lösung, z.B. Natriumhydroxid-Lösung, sowie eines Phasentransferkatalysators, z.B. Tetrabutylammoniumhydrogensulfat, erfolgen.

Typische Reaktionsbedingungen können den Beispielen entnommen werden.

Falls die Verbindungen der Formel I in verschiedenen Stereoisomeren auftreten können, betrifft die Erfindung sowohl die reinen Isomeren als auch alle möglichen Isomerengemische.

Neue für die Herstellung der Verbindungen der Formel I verwendete Ausgangs- und Zwischenprodukte, ihre Verwendung und Verfahren zu ihrer Herstellung bilden ebenfalls einen Gegenstand der Erfindung.

Von besonderer Bedeutung sind dabei
(1) das Verfahren zur Herstellung einer Verbindung der Formel la, 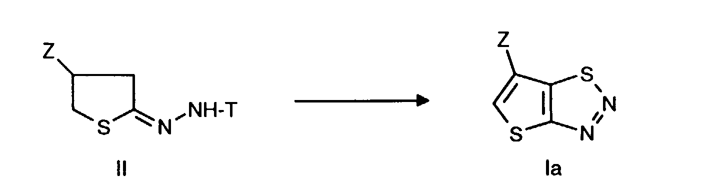 worin Z die für Formel I angegebenen Bedeutungen hat, dadurch gekennzeichnet, dass man eine Verbindung der Formel II, worin T eine Gruppe CO-Y, CONY₂ oder SO₂-Y und Y Wasserstoff, gegebenenfalls substituiertes C₁-C₈₋-Alkyl oder gegebenenfalls substituiertes Aryl bedeuten oder T eine Gruppe COO-U und U ein gegebenenfalls substituiertes C₁-C₈₋-Alkyl oder gegebenenfalls substituiertes Aryl bedeutet, mit Thionylchlorid umsetzt.
(2) das Verfahren zur Herstellung einer Verbindung der Formel I, 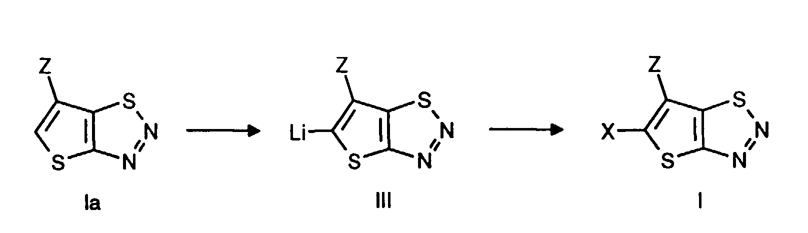 worin X und Z die für Formel I angegebenen Bedeutungen haben, dadurch gekennzeichnet, dass man eine Verbindung der Formel la, worin Z die für Formel I angegebenen Bedeutungen hat, mit einer lithiumorganischen Verbindung wie z.B.Butyllithium, sec.Butyllithium, Hexyllithium, Lithiumdiisopropylamid (LDA), Lithiumhexamethyldisilazid oder Lithiumtetramethylpiperidid (LTMP) in einem aprotischen Lösungsmittel wie z.B. THF, Diethylether oder Hexan in An- oder Abwesenheit eines zusätzlichen Komplexierungsmittels bei -100°C bis +20°C zu einer lithiumorganischen Verbindung der Formel III und diese weiterumsetzt
   a) zur Herstellung einer Verbindung, worin X Halogen bedeutet, mit einem Halogen;
   b) zur Herstellung einer Verbindung, worin X Chlor bedeutet, mit Hexachlorethan;
   c) zur Herstellung einer Verbindung, worin X C₁-C₄-Alkyl bedeutet, mit einem C₁-C₄-Alkylhalogenid;
   d) zur Herstellung einer Verbindung, worin X CH₂OH bedeutet, mit Formaldehyd;
   e) zur Herstellung einer Verbindung, worin X CHO bedeutet, mit Dimethylformamid.;
   f) zur Herstellung einer Verbindung, worin X COOR₈ bedeutet, mit CO₂ und gegebenenfalls anschliessend verestert.
(3) das Verfahren zur Herstellung einer lithiumorganischen Verbindung der Formel III 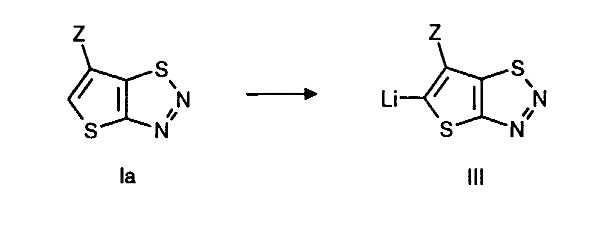 worin Z die für Formel I angegebenen Bedeutungen hat, dadurch gekennzeichnet, dass man eine Verbindung der Formel la, worin Z die für Formel I angegebenen Bedeutungen hat, mit einer lithiumorganischen Verbindung wie z.B.Butyllithium, sec.Butyllithium, Hexyllithium, Lithiumdiisopropylamid (LDA), Lithiumhexamethyldisilazid oder Lithiumtetramethylpiperidid (LTMP) in einem aprotischen Lösungsmittel wie z.B. THF, Diethylether oder Hexan in An- oder Abwesenheit eines zusätzlichen Komplexierungsmittels bei -100°C bis +20°C zu einer lithiumorganischen Verbindung der Formel III umsetzt, sowie
(4) das Verfahren zur Herstellung einer Verbindung der Formel I 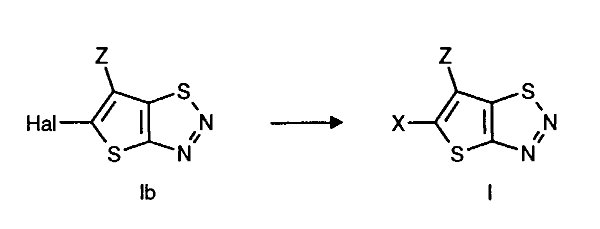 worin Z die für Formel I angegebenen Bedeutungen hat und X SR₈, oder N(R₉)R₁₀ bedeuten, dadurch gekennzeichnet, dass man eine Verbindung der Formel Ib, worin Hal Halogen bedeutet, mit HSR₈, oder HN(R₉)R₁₀ unter basischen Bedingungen umsetzt.

Bevorzugte Zwischenprodukte sind die Verbindungen der Formel II 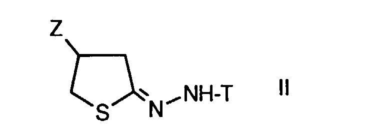 worin Z die für Formel I angegebenen Bdeutungen hat und T eine Gruppe CO-Y, CONY₂ oder SO₂-Y und Y Wasserstoff, gegebenenfalls substituiertes C₁-C₈₋-Alkyl oder gegebenenfalls substituiertes Aryl bedeuten, oder T eine Gruppe COO-U und U ein gegebenenfalls substituiertes C₁-C₈₋-Alkyl oder gegebenenfalls substituiertes Aryl, insbesondere aber COO-C₁-C₄-Alkyl bedeutet;

ferner lithiumorganische Verbindungen der Formel III 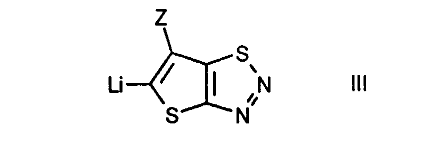 worin Z die für Formel I angegebenen Bedeutungen hat, als Lösung in einem aprotischen Lösungsmittel.

Thieno-1,2,3-thiadiazole sind in EP-A-138,622 generisch beschrieben; in dieser Referenz ist allerdings weder ein Beispiel noch eine Herstellungsmethode angegeben. Ferner ist das 6-Methyl-5-ethoxycarbonyl-thieno[2,3-d]-1,2,3-thiadiazol X 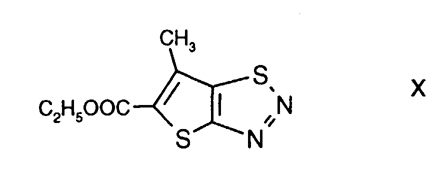 ohne Angabe irgendeiner Verwendung in J. prakt.Chem. 330, S.866-872, (1988) beschrieben; dieses unterscheidet sich durch die Art der Substituenten von den Verbindungen der vorliegenden Erfindung.

Verbindungen der Formel I sind neu und haben eine unerwartet hohe mikrobizide Wirksamkeit. Dabei kann der Schutz der Pflanzen sowohl durch direkte Einwirkung auf die Schädlinge als auch durch Aktivierung und Stimulierung des pflanzeneigenen Abwehrsystems eintreten (Immunisierung). Diese Wirkungsweise ist auch unter der Bezeichnung "Systemic Activated Disease Resistance", ("SAR") bekannt geworden. Durch die Anwendung der erfindungsgemässen Verbindungen kann somit eine Gesunderhaltung und Stärkung der Pflanzen aus eigener Kraft erreicht werden.

Die Verbindungen der Formel I lassen sich auf dem Agrarsektor und verwandten Gebieten präventiv und/oder kurativ als Wirkstoffe bei der Bekämpfung von Pflanzenschädlingen einsetzen. Die erfindungsgemässen Wirkstoffe der Formel I zeichnen sich durch hervorragende Wirkung auch bei niedrigen Anwendungskonzentrationen, durch gute Pflanzenverträglichkeit und Umweltfreundlichkeit aus. Sie besitzen sehr vorteilhafte, insbesondere systemische, Eigenschaften und können zum Schutz von zahlreichen Kulturpflanzen eingesetzt werden. Mit den Wirkstoffen der Formel I können an Pflanzen oder Pflanzenteilen (Früchten, Blüten, Laubwerk, Stengeln, Knollen, Wurzeln) von unterschiedlichen Nutzkulturen die auftretenden Schädlinge eingedämmt oder vernichtet werden, wobei auch später zuwachsende Pflanzenteile, z. B. von phytopathogenen Mikroorganismen, verschont bleiben.

Die Verbindungen I können ferner als Beizmittel zur Behandlung von Saatgut (Früchten, Knollen, Körnem) und Pflanzenstecklingen zum Schutz vor Pilzinfektionen sowie gegen im Erdboden auftretende phytopathogene Pilze eingesetzt werden.

Die Verbindungen I sind z. B. gegen die den folgenden Klassen angehörenden phytopathogenen Pilze wirksam: Fungi imperfecti (z. B. Botrytis, Pyricularia, Helminthosporium, Fusarium, Septoria, Cercospora und Alternaria) und Basidiomyceten (z. B. Rhizoctonia, Hemileia, Puccinia). Darüber hinaus wirken sie gegen die Klassen der Ascomyceten (z. B. Venturia und Erysiphe, Podosphaera, Monilinia, Uncinula) und der Oomyceten (z. B. Phytophthora, Pythium, Plasmopara).

Als Zielkulturen für den pflanzenschützenden Einsatz gelten im Rahmen der Erfindung beispielsweise folgende Pflanzenarten: Getreide (Weizen, Gerste, Roggen, Hafer, Reis, Mais, Sorghum und verwandte Spezies); Rüben (Zucker- und Futterrüben); Kern-, Stein und Beerenobst (Aepfel, Bimen, Pflaumen, Pfirsiche, Mandeln, Kirschen, Erdbeeren, Himbeeren und Brombeeren); Hülsenfrüchte (Bohnen, Linsen, Erbsen, Soja); Oelkulturen (Raps, Senf, Mohn, Oliven, Sonnenblumen, Kokos, Rizinus, Kakao, Erdnüsse); Gurkengewächse (Kürbis, Gurken, Melonen); Fasergewächse (Baumwolle, Flachs, Hanf, Jute); Citrusfrüchte (Orangen, Zitronen, Pampelmusen, Mandarinen); Gemüsesorten (Spinat, Kopfsalat, Spargel, Kohlarten, Möhren, Zwiebeln, Tomaten, Kartoffeln, Paprika); Lorbeergewächse (Avocado, Cinnamonium, Campfer) und Pflanzen wie Tabak, Nüsse, Kaffee, Eierfrüchte, Zuckerrohr, Tee, Pfeffer, Reben, Hopfen, Bananen- und Naturkautschukgewächse sowie Zierpflanzen.

Wirkstoffe I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können z. B. Düngemittel, Spurenelemente-Vermittler oder andere das Pflanzenwachstum beeinflussende Präparate sein. Es können dabei auch selektive Herbizide sowie Insektizide, Fungizide, Bakterizide, Nematizide, Molluskizide oder Gemische mehrerer dieser Präparate zusammen mit gegebenenfalls weiteren in der Formulierungstechnik üblichen Trägerstoffen, Tensiden oder anderen applikationsfördernden Zusätzen Verwendung finden.

Geeignete Träger und Zusätze können fest oder flüssig sein und sind in der Formulierungstechnik zweckdienliche Stoffe, z. B. natürliche oder regenerierte mineralische Stoffe, Lösungs-, Dispergier-, Netz-, Haft-, Verdickungs-, Binde- oder Düngemittel.

Ein bevorzugtes Verfahren zum Aufbringen eines Wirkstoffs der Formel I bzw. eines agrochemischen Mittels, das mindestens einen dieser Wirkstoffe enthält, ist das Aufbringen auf das Blattwerk (Blattapplikation). Applikationsfrequenz und Aufwandmenge richten sich dabei nach dem Befallsdruck des betreffenden Erregers. Die Wirkstoffe I können aber auch über den Erdboden durch das Wurzelwerk in die Pflanze gelangen (systemische Wirkung), indem man den Standort der Pflanze mit einer flüssigen Zubereitung tränkt oder die Substanzen in fester Form in den Boden einbringt, z. B. in Form von Granulat (Bodenapplikation). Bei Wasserreiskulturen kann man solche Granulate dem überfluteten Reisfeld zudosieren. Die Verbindungen I können aber auch zur Saatgutbehandlung auf Samenkömer aufgebracht werden (Coating), indem man die Kömer bzw. Knollen entweder in einer flüssigen Zubereitung des Wirkstoffs tränkt oder sie mit einer festen Zubereitung beschichtet.

Die Verbindungen I werden dabei in unveränderter Form oder vorzugsweise zusammen mit den in der Formulierungstechnik üblichen Hilfsmitteln eingesetzt. Dafür werden sie zweckmässigerweise z. B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln oder Granulaten, z. B. durch Verkapselung in, z. B. polymeren, Stoffen, in bekannter Weise verarbeitet. Die Anwendungsverfahren wie Versprühen, Vernebeln, Verstäuben, Verstreuen, Bestreichen oder Giessen werden ebenso wie die Art der Mittel den angestrebten Zielen und den gegebenen Verhältnissen entsprechend gewählt.

Günstige Aufwandmengen liegen im allgemeinen bei 5 g bis 2 kg Aktivsubstanz (AS) je Hektar (ha), bevorzugt bei 10 g bis 1 kg AS/ha, insbesondere bei 20 g bis 600 g AS/ha. Bei der Verwendung als Saatbeizmittel werden mit Vorteil Dosierungen von 10 mg bis 1 g Aktivsubstanz pro kg Saatgut verwendet.

Die Formulierungen, d. h. die den Wirkstoff der Formel I und gegebenenfalls einen festen oder flüssigen Zusatzstoff enthaltenden Mittel, Zubereitungen oder Zusammensetzungen, werden in bekannter Weise hergestellt, z. B. durch inniges Vermischen und/oder Vermahlen des Wirkstoffs mit Streckmitteln, wie Lösungsmitteln, festen Trägerstoffen und gegebenenfalls oberflächenaktiven Verbindungen (Tensiden).

Als Lösungsmittel kommen in Frage: Aromatische Kohlenwasserstoffe, bevorzugt die Fraktionen C₈ bis C₁₂, wie Xylolgemische oder substituierte Naphthaline, Phthalsäureester, wie Dibutyl- oder Dioctylphthalat, aliphatische Kohlenwasserstoffe, wie Cyclohexan oder Paraffine, Alkohole und Glycole sowie deren Ether und Ester, wie Ethanol, Ethylenglycol, Ethylenglycolmonomethyl- oder -ethylether, Ketone, wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl, und Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen, wie Bimsstein, Ziegelbruch, Sepiolit oder Bentonit, als nicht-sorptive Trägermaterialien z. B. Calcit oder Sand in Frage. Darüber hinaus kann eine Vielzahl von vorgranulierten Materialien anorganischer oder organischer Natur, wie Dolomit oder zerkleinerte Pflanzenrückstände, verwendet werden.

Als oberflächenaktive Verbindungen kommen je nach Art des zu formulierenden Wirkstoffs der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sogenannte wasserlösliche Seifen, als auch wasserlösliche synthetische oberflächenaktive Verbindungen sein.

Als Beispiele nichtionischer Tenside seien Nonylphenolpolyethoxyethanole, Ricinusölpolyglycolether, Polypropylen-/Polyethylen-oxidaddukte, Tributylphenoxypoly ethylenethanol, Polyethylenglycol und Octylphenoxypolyethoxyethanol erwähnt.
Femer kommen auch Fettsäureester von Polyoxyethylensorbitan, wie das Polyoxyethylensorbitan-trioleat, in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen.

Weitere in der Formulierungstechnik gebräuchlichen Tenside sind dem Fachmann bekannt oder können der einschlägigen Fachliteratur entnommen werden.

Die agrochemischen Zubereitungen enthalten in der Regel 0,1 bis 99 Gewichtsprozent, insbesondere 0,1 bis 95 Gewichtsprozent, Wirkstoff der Formel 1, 99,9 bis 1 Gewichtsprozent, insbesondere 99,8 bis 5 Gewichtsprozent, eines festen oder flüssigen Zusatzstoffes und 0 bis 25 Gewichtsprozent, insbesondere 0,1 bis 25 Gewichtsprozent, eines Tensids.

Während als Handelsware eher konzentrierte Mittel bevorzugt werden, verwendet der Endverbraucher in der Regel verdünnte Mittel.

Die Mittel können auch weitere Zusätze, wie Stabilisatoren, Entschäumer, Viskositätsregulatoren, Bindemittel oder Haftmittel, sowie Dünger oder andere Wirkstoffe zur Erzielung spezieller Effekte enthalten.

### Beispiele:

### 1. Herstellungsbeispiele

Abkürzungen: Min. = Minute, RG = Reaktionsgemisch, RT = Raumtemperatur, Smp. = Schmelzpunkt, Std. = Stunde, THF = Tetrahydrofuran

### Beispiel 1:

### Herstellung von Acetylthiomethylbutandisäure (VII): (Zwischenprodukt)

Zu 280 g (3.68 mol) Thioessigsäure werden unter gleichzeitigem Erwärmen des Heizbades auf 95°C innert ca. 30 Min. 400 g (3.07 mol) Itaconsäure (VIII) portionenweise zugegeben. Nach 2.5 Std. rühren bei 90 - 100°C wird das RG auf RT abgekühlt, mit 300 ml Diisopropylether (DIPE) verdünnt, worauf das Produkt innert ca. 12 Std. auskristallisiert; man erhält 582 g Kristalle vom Smp. 89-91°C.

### Beispiel 2: Herstellung von Mercaptomethylbutandisäure (VI): (Zwischenprodukt)

Zu einer Lösung von 570 g (14.25 mol) NaOH in 3 1 Wasser werden unter Stickstoff und Eiskühlung 582 g (2.85 mol) Acetylthiomethylbutandisäure (VII) gegeben (exotherm). Nach 15 Std. rühren bei RT wird das RG mit konzentrierter Salzsäure auf pH1 angesäuert und mit Ether extrahiert. Die vereinigten organischen Phasen werden über Natriumsulfat getrocknet, zur Trockene eingeengt, und der kristalline Rückstand mit 200 ml kaltem Dichlormethan gewaschen. Man erhält 385 g Kristalle vom Smp. 107-108.5°C.

### Beispiel 3: Herstellung von Tetrahydro-5-oxo-3-thiophencarbonsäure (IVa) (Zwischenprodukt)

Eine Schmelze von 385 g (2.34 mol) Mercaptomethylbutandisäure (Vl) wird während 3 Std. auf 140-150°C erhitzt. Nach abkühlen auf RT werden 315 g erstarrtes Produkt vom Smp. 106-108°C erhalten.

### Beispiel 4

### Herstellung von Tetrahydro-5-oxo-3-thiophencarbonsäuremethylester: (Zwischenprodukt)

Zu einer Lösung von 160 g (1.095 mol) Tetrahydro-5-oxo-3-thiophencarbonsäure, 70 g (2.19 mol) Methanol und 4 g (32.8 mmol) Dimethylaminopyridin (DMAP) in 2.5 Diethylether werden unter Eiskühlung 225 g (1.09 mol) Dicyclohexylcarbodiimid (DCC) portionenweise zugegeben. Nach 4 Std. rühren bei RT wird der Dicyclohexylharnstoff abfiltriert, das Filtrat mit je 500 ml 2N Salzsäure und gesättigter Natriumhydrogenkarbonatlösung extrahiert, mit Wasser gewaschen und über Natriumsulfat getrocknet. Es werden 149 g festes Produkt vom Smp. 33-35°C erhalten.

### Beispiel 5

### Herstellung von Tetrahydro-5-thioxo-3-thiophencarbonsäuremethylester: (Zwischenprodukt)

Zu einer Suspension von 149 g (0.93 mol) Tetrahydro-5-oxo-3-thiophencarbonsäuremethylester in 1.23 1 absolutem Toluol werden bei RT 207 g (0.51 mol) Lawesson-Reagens gegeben und 16 Std. am Rückfluss gekocht. Danach wird das Lösungsmittel im Vakuum abdestilliert, der Rückstand viermal in je 1 l eines Lösungsmittelgemisches aus Petrolether und Diethylether 1:1 20 Min. am Rückfluss gekocht und die Extraktphase jeweils abdekantiert und eingeengt. Nach Kugelrohrdestillation der Rohsubstanz bei 130-140°C/0.03 mbar werden 129.5 g oranges Öl erhalten.

### Beispiel 6

### Herstellung von 5-(2-Ethoxycarbonyl-2-hydrazinyl-1-yliden)-tetrahydro-3-thiophencarbonsäuremethylester (IIa): (Zwischenprodukt)

Zu einer Lösung von 253 g (1.43 mol) Tetrahydro-5-thioxo-3-thiophencarbonsäuremethylester in absolutem Ethanol werden bei RT portionenweise 149 g (1.43 mol) Hydrazincarbonsäureethylester gegeben und 4 Std. am Rückfluss gekocht. Das RG wird bis zur Trockene eingeengt und der Rückstand aus 200 ml Isopropanol umkristallisiert. Man erhält 315.6 g kristallines Produkt vom Smp. 89-91°C.

### Beispiel 7

### Herstellung von Thieno[2,3-d]1,2,3-thiadiazol-6-carbonsäuremethylester

Zu einer Lösung von 315.6 g (1.28 mol) 5-(2-Ethoxycarbonyl-2-hydrazinyl-1-yliden)-tetrahydro-3-thiophencarbonsäuremethylester in 2 1 Dichlormethan werden unter Eiskühlung langsam 3044 g (25.6 mol) Thionylchlorid zugetropft derart, dass das Lösungsmittel nicht zu sieden beginnt. Danach wird das RG 16 Std. bei RT gerührt, das Lösungsmittel und überschüssiges Thionylchlorid abdestilliert, der Rückstand 30 Min. mit 2 1 Ethylacetat digeriert und der Festkörper nach Entfernen des Lösungsmittels umkristallisiert. Man erhält 155.3 g kristallines Produkt vom Smp. 139-141°C, das noch ca. 4% 5-Chlorthieno[2,3-d]1,2,3-thiadiazol-6-carbonsäuremethylester als Nebenprodukt enthält.

### Beispiel 8

### Herstellung von Thieno[2,3-d]1,2,3-thiadiazol-6-carbonsäure

Zu einer Suspension von 145 g (0.724 mol) Thieno[2,3-d]1,2,3-thiadiazol-6-carbonsäuremethylester (la) in Ethanol wird eine Lösung von 58 g (1.45 mol) NaOH in 400 ml Wasser gegeben und 90 Min. bei RT gerührt. Danach wird das Ethanol weitgehend abdestilliert, die verbleibende Lösung unter Eiskühlung mit 130 ml konzentrierter Salzsäure angesäuert, das Produkt abfiltriert, mit Wasser gewaschen und getrocknet. Man erhält 131.5 g kristallines Produkt vom Smp. 268-272 °C (Zersetzung).

### Beispiel 9

### Herstellung von 5-Chlorthieno[2,3-d]1,2,3-thiadiazol-6-carbonsäure:

Zu einer Lösung von 20.4 g (201.6 mmol) Diisopropylamin in 200 ml THF werden bei -30°C unter Stickstoff 79 ml (201.6 mmol) n-Butyllithium (1.6 M in Hexan) gegeben. Nach 20 Min. rühren bei -20°C wird die so hergestellte LDA-Lösung bei -90°C unter Stickstoff zu einer Suspension von 15.0 g (80.6 mmol) Thieno[2,3-d]1,2,3-thiadiazol-6-carbonsäure in 150 ml THF mittels Kapillare zugetropft. Nach 90 Min. rühren bei -50°C wird bei -90°C eine Lösung von 42.0 g (177.2 mmol) Hexachlorethan in 70 ml THF zugegeben und das RG innerhalb von zwei Std. auf RT erwärmt. Danach wird das RG auf 2 1 Wasser gegossen, die wässrige Phase mit Diethylether extrahiert, mit konzentrierter Salzsäure angesäuert und das Produkt abfiltriert. Man erhält 17.55 g helbraunen Feststoff vom Smp. 255-265 (Zersetzung).

### Beispiel 10

### Herstellung von 5-Chlorthieno[2,3-d]1,2,3-thiadiazol-6-carbonsäuremethylester:

Zu einer Lösung von 54.28 g (246 mmol) 5-Chlorthieno[2,3-d]1,2,3-thiadiazol-6-carbonsäure, 39.40 g (1.23 mol) Methanol und 2.00g (16.4 mmol) 4-Dimethylaminopyridin (DMAP) in 1 l Dichlormethan werden bei RT unter kräftigem Rühren 50.70 g (246 mmol) Dicyclohexylcarbodiimid (DCC) in kleinen Portionen zugegeben. Nach 16 Std. rühren wird der Dicyclohexylharnstoff abfiltriert und mit Dichlormethan gewaschen. Die vereinigten organischen Phasen werden je einmal mit 2N Salzsäure, gesättigter Natriumhydrogencarbonatlösung und Wasser extrahiert, über Natriumsulfat getrocknet und zur Trockene eingeengt. Der Rückstand wird mit Dichlormethan/Petrolether 1:1 über 500 g Kieselgel 60 chromatographiert. Man erhält 45.3 g eines farblosen Feststoffs vom Smp. 124-126 °C.

### Beispiel 11 Herstellung von Thieno[2,3-d]1,2,3-thiadiazol und 5-Chlorthieno[2,3-d]1,2,3-thiadiazol der Formel Ia1 resp. Ik

Eine Lösung von 9.5 g 5-(2-Ethoxycarbonyl-2-hydrazinyl-1-yliden)-tetrahydrothiophen in 100 ml Dichlormethan wird unter Stickstoffatmosphäre und Rühren bei Raumtemperatur tropfenweise mit 20 ml Thionylchlorid, gelöst in 20 ml Dichlormethan, versetzt, wobei sich die Innentemperatur nach kurzer Zeit auf ca. 35°C erhöht und Abgang von gasförmiger Salzsäure einsetzt. Ueber Nacht wird bei Raumtemperatur und dann noch 3 Stunden unter Rückfluss gerührt, eingedampft, der Rückstand zwischen Essigester und Eiswasser verteilt, die organischen Extrakte mit Wasser gewaschen, getrocknet und eingedampft. Der Rückstand wird an Kieselgel (Hexan-Essigsäureethylester 9:1) gereinigt, wobei der Reihe nach 5-Chlorthieno(2,3-d)1,2,3-thiadiazol la1 vom Smp. 117-119°C und als Hauptprodukt Thieno (2.3-d)1,2,3-thiadiazol la2 mit Smp. 78-79°C erhalten werden.

Die Umsetzung kann auch in anderen Lösungsmitteln wie z.B. Toluol vorgenommen werden; zudem können die Reaktanden invers zur Reaktion gebracht werden indem z.B. eine auf 70°C erwärmte Lösung des Thionylchlorids in Toluol bei dieser Temperatur tropfenweise mit der toluolischen Lösung des Carbazons versetzt und bis zur völligen Umsetzung gerührt werden kann.

### Beispiel 12 (Zwischenprodukt)

### Herstellung von 5-(2-Ethoxycarbonyl-2-hydrazinyl-1-yliden)-tetrahydrothiophen

Eine Lösung von 20,5 g Ethylcarbazat gelöst in 170 ml warmem Ethanol wird zu einer Lösung von 23 g Tetrahydro-2-thioxothiophen gegeben und bis zur völligen Umsetzung unter Rückfluss gekocht, wobei Schwefelwasserstoff entweicht. Dann wird eingedampft und der Rückstand durch Zugabe von Essigester und Hexan zur Kristallisation gebracht. Der Niederschlag wird abfiltriert, mit Hexan gewaschen und getrocknet, wobei die Titelverbindung mit Smp. 55-57°C resultiert.

### Beispiel 13 (Zwischenprodukt)

### Herstellung von 2-Amino-4-methoxymethylthiophen-5-carbonsäuremethylester-Hydrochlorid

Ein Gemisch aus 355 g 3-Methoxymethyl-5-cyano-pent-3-en-carbonsäuremethylester (hergestellt analog J. prakt. Chem. 315, 542 (1973)), 67,2 g Schwefel in 1 l Ethanol werden Rühren und Kühlen bei max 25°C tropfenweise mit 217 ml Diethylamin versetzt und über Nacht bei Raumtemperatur gerührt. Dann wird abgekühlt und bei max. 22°C 1575 ml Salzsäure konz. zugetropft. Nach weiterem Rühren bei -5°C entsteht ein Niederschlag, welcher abfiltriert, mit Ether gewaschen und aus Dioxan umkristallisiert wird. Farblose Kristalle mit Smp. 157-158°C.

### Beispiel 14 Herstellung von 6-Methoxymethyl-thieno[2,3-d]-1,2,3-thiadiazol-5-carbonsäuremethylester

In einem Sulfierkolben werden unter Rühren und Kühlen 20 ml Eiswasser mit 11 ml Schwefelsäure (konz. 96 %) versetzt und bei -5°C 5.16 g 2-Amino-3-thiocyanato-4-methoxymethyl-5-carbonsäuremethylester (hergestellt analog J. prakt. Chem. 315, 542 (1973)), portionenweise eingetragen, die Suspension mit 10 ml Dioxan verdünnt und anschliessend bei -3° - 0°C eine Lösung von 1.656 g Natriumnitrit in 2,8 ml Wasser unter die Oberfläche zudosiert und noch 3/4 h bei 0°C weitergerührt. Dann wird mit Dichlormethan extrahiert, die Extrakte mit Wasser gewaschen und getrocknet, filtriert und eingedampft. Das verbleibende Oel wird an Kieselgel gereinigt wobei die Titelverbindung mit Smp. 123-124°C resultiert.

### Beispiel 15 (Zwischenprodukt)

### Herstellung von Thiomethoxycarbonyl-2-thienyl-thiohamstoff

Eine Lösung aus 3,9 g 2-Amino-4-thiophencarbonsäure-methylester in 25 ml Chlorbenzol wird auf -5°C abgekühlt und bei dieser Temperatur vorsichtig mit 0,7 ml Schwefelsäure konz. (96 %) und 2,25 g Natriumrhodanid versetzt und 15 Minuten bei 0°C und anschliessend 5 Stunden bei 100°C gerührt. Dann wird abgekühlt, in warmem Tetrahydrofuran aufgenommen, über Kieselgel filtriert und eingedampft. Das gewünschte Produkt hat nach Reinigung über Kieselgel (Hexan-Essigsäureethylester 1:1) einen Smp. vom 165-166°C.

### Beispiel 16 (Zwischenprodukt)

### Herstellung von 2-Amino-thieno[2,3-d]thiazol-6-carbonsäuremethylester

Zu 4,0 g Thiomethoxycarbonyl-2-thienyl-thiohamstoff in 50 ml Chlorbenzol werden bei-10°C innert 30 Minuten unter starkem Rühren 0,97 ml Brom in 40 ml Chlorbenzol zugetropft. Dann wird während 1 Stunde gegen Raumtemperatur gerührt und noch 1/2 Stunden bei 65°C gehalten. Dann wird abgekühlt, der Niederschlag abfiltriert, mit wenig Diethylether gewaschen und mit 25 ml Natriumhydrogencarbonat (NaHCO₃; gesättigte Lösung) verrieben, wieder filtriert und mit Wasser gewaschen. Der Niederschlag wird in warmem Tetrahydrofuran aufgenommen, über Natriumsulfat getrocknet, filtriert und das Filtrat eingedampft. Der Rückstand wird an Kieselgel (Hexan-Essigsäureethylester 2:3 gereinigt wobei reines Produkt vom Smp. 221-223°C erhalten wird.

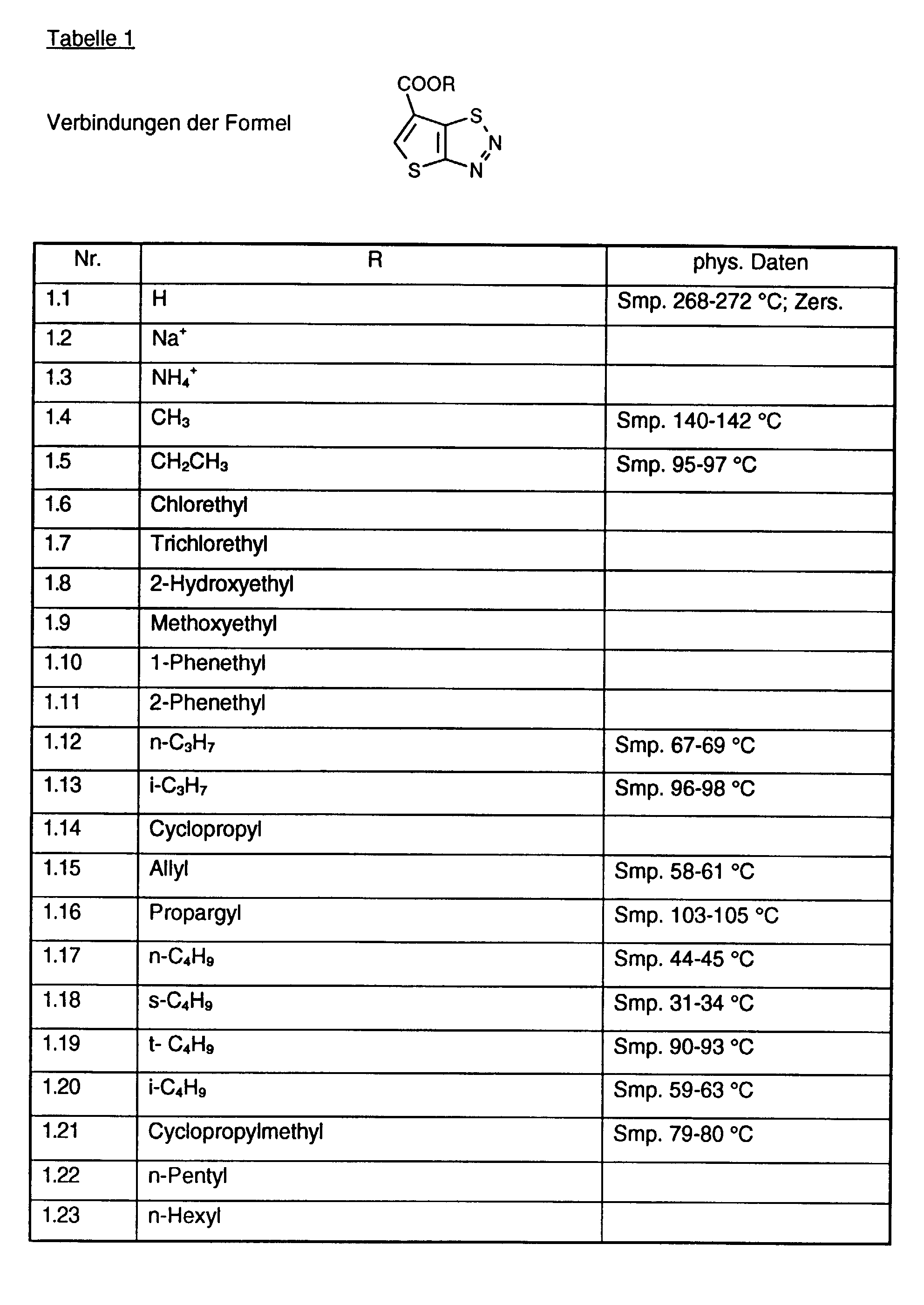

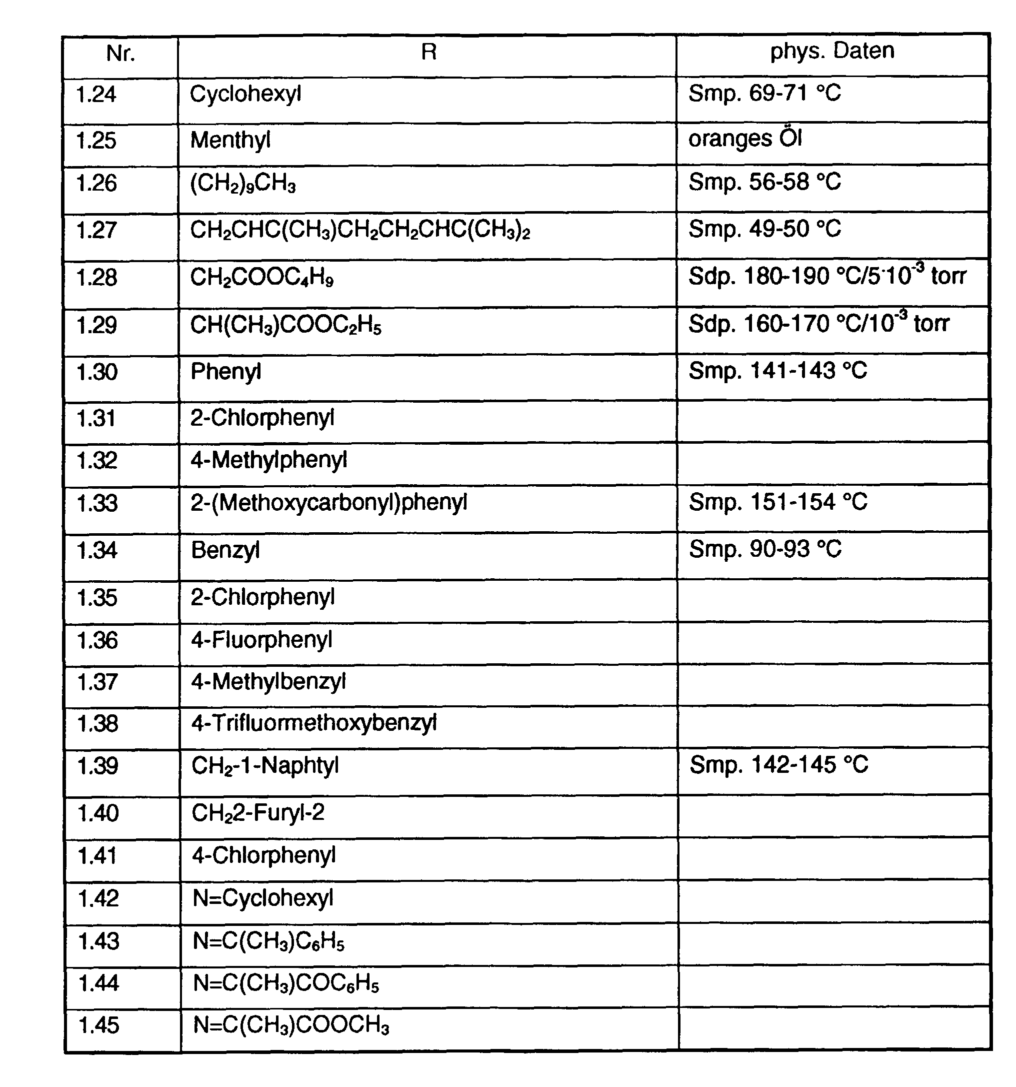

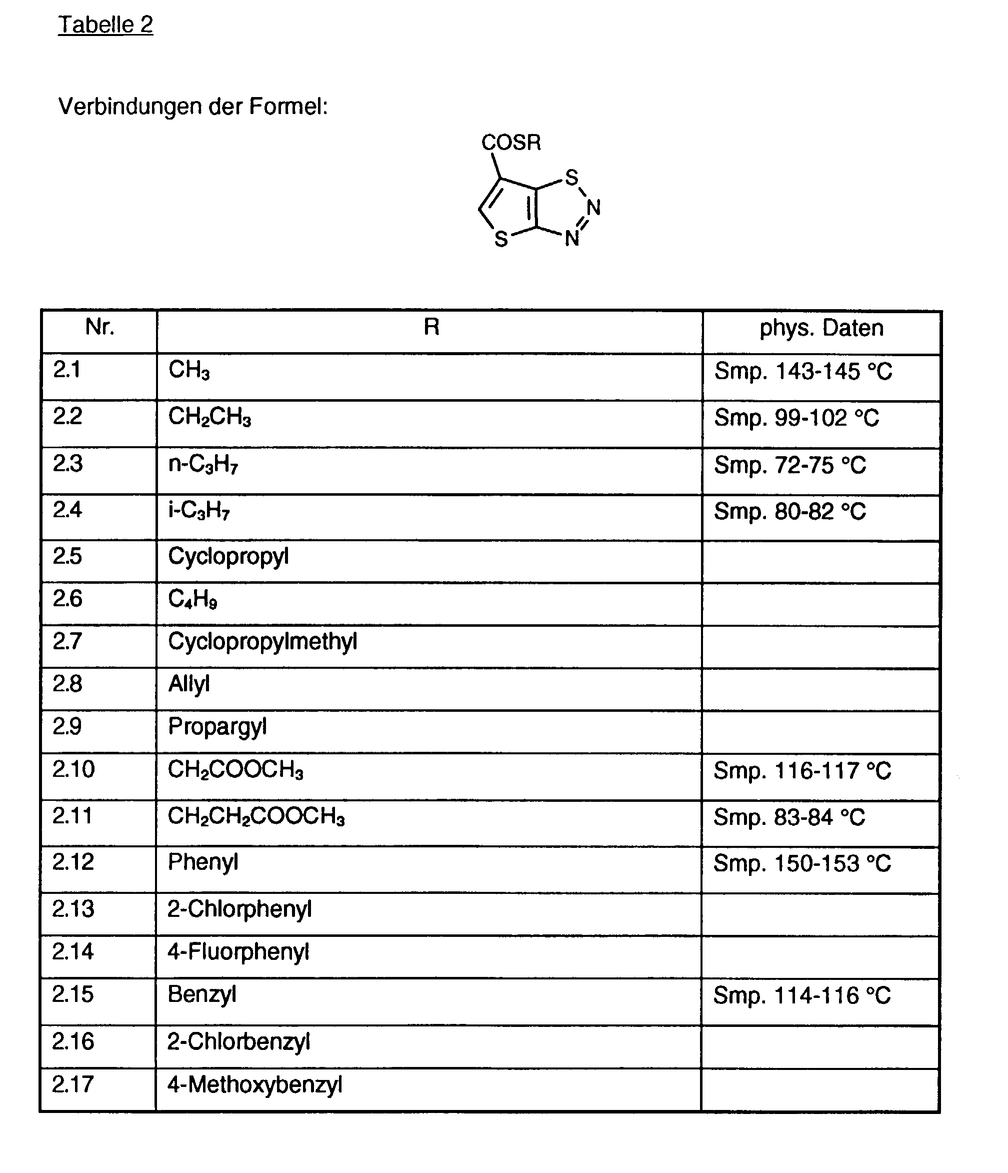

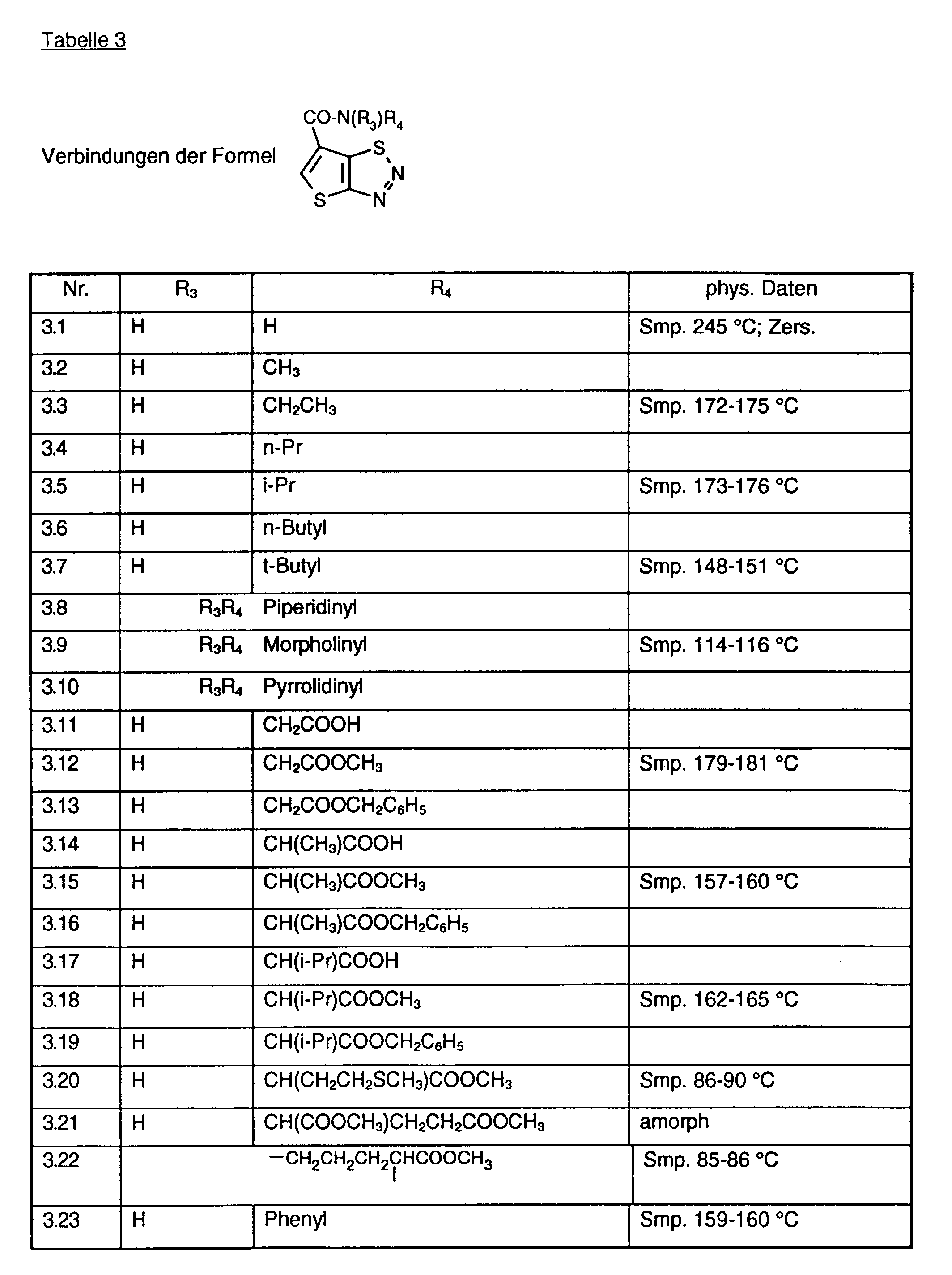

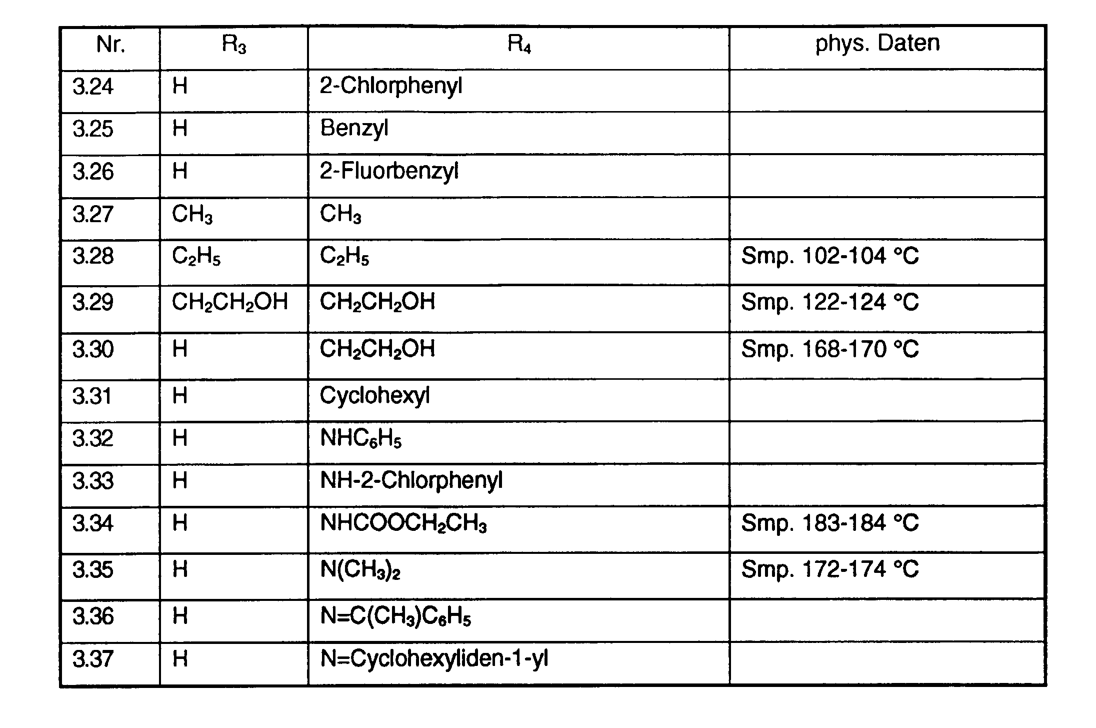

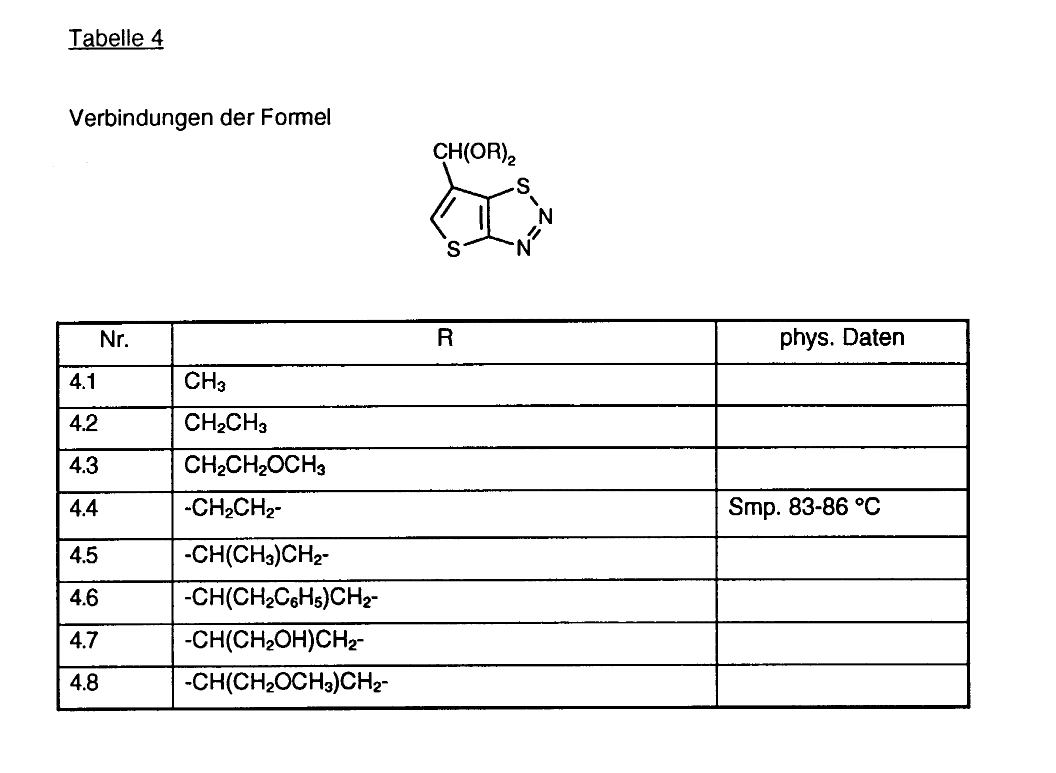

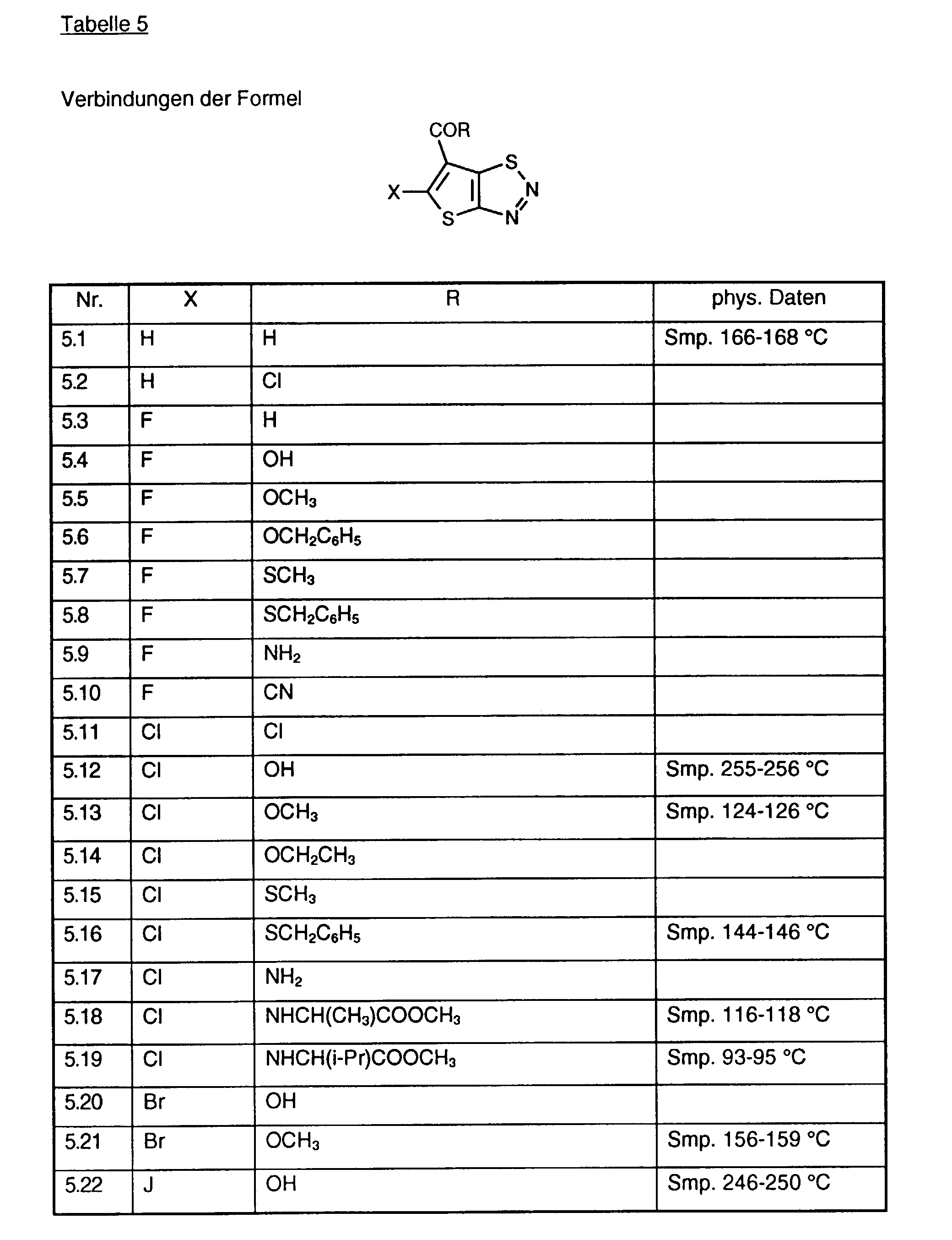

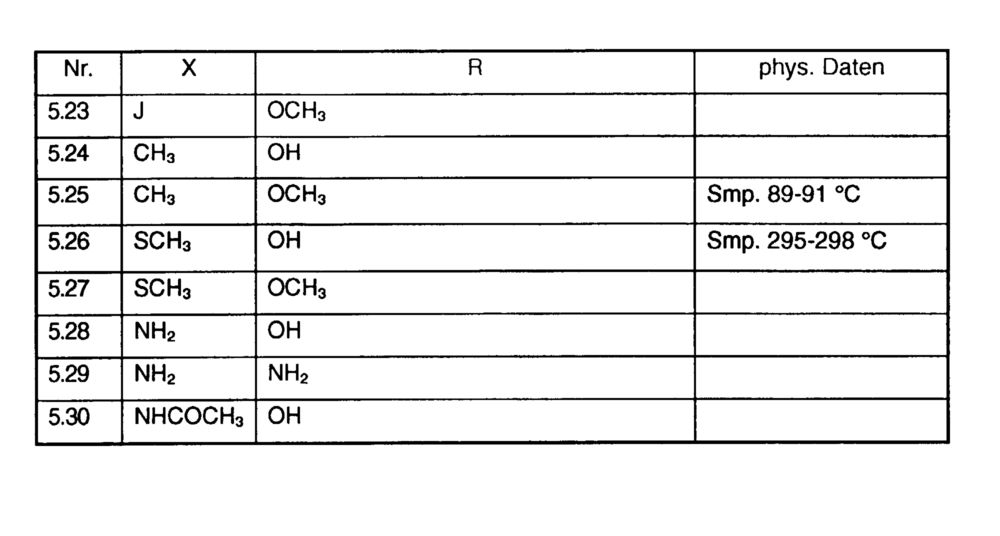

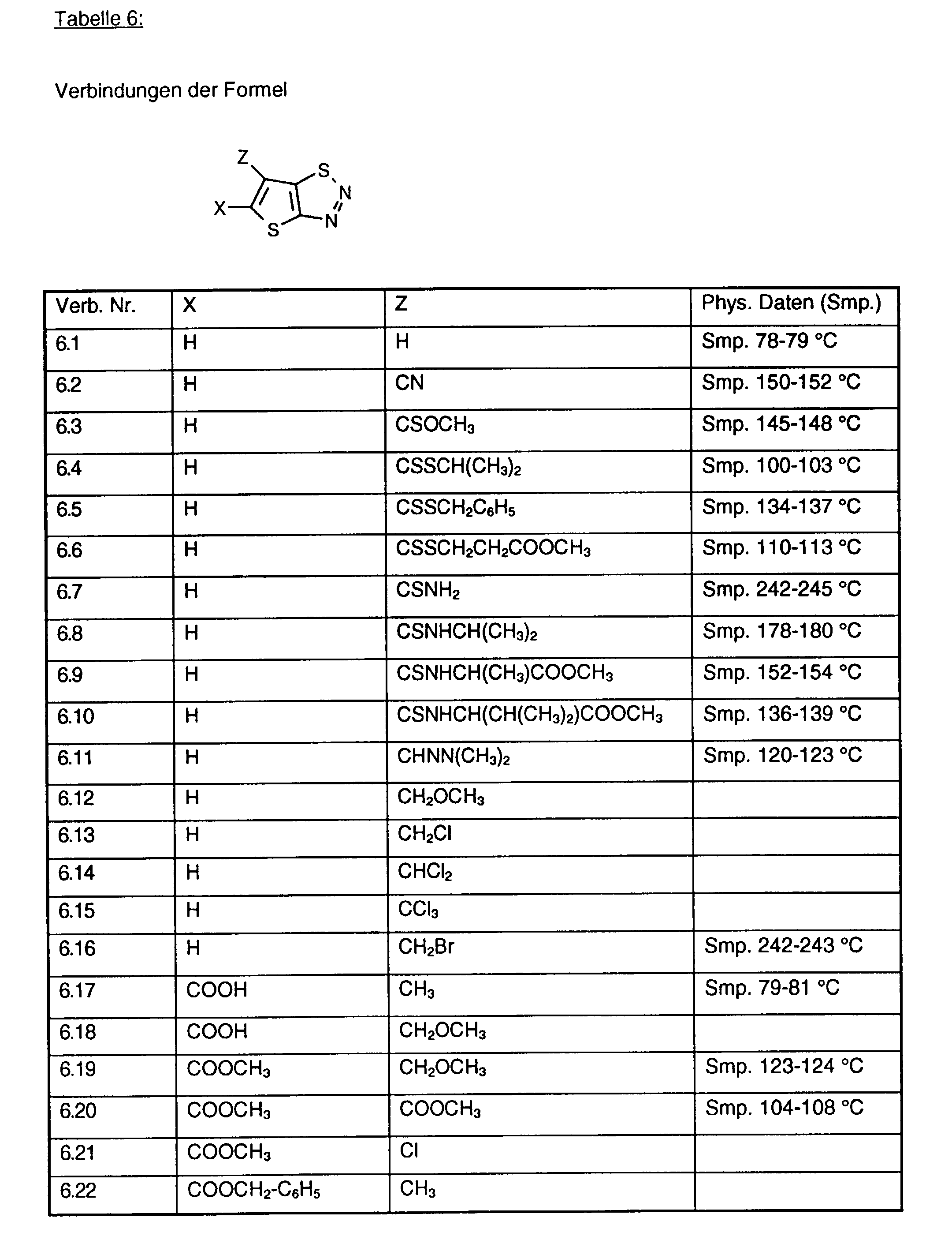

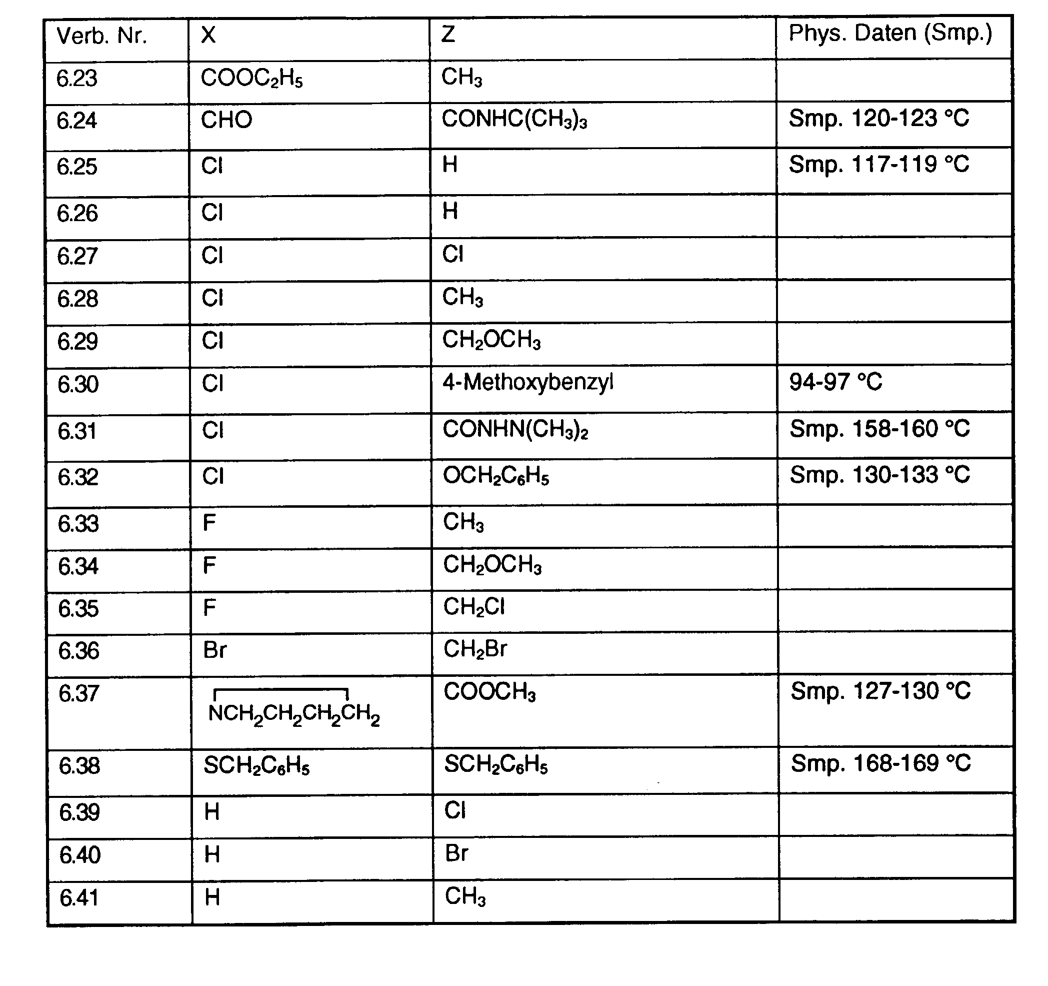

### 2. Formulierungsbeispiele für Wirkstoffe aus den Tabellen (% = Gewichtsprozent)

| 2.1 Spritzpulver | a) | b) | c) |
|---|---|---|---|
| Wirkstoff aus den Tabellen | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyethylenglykolether (7-8 Mol Ethylenoxid) | - | 2 % | - |
| Hochdisperse Kieselsäure | 5 % | 10 % | 10 % |
| Kaolin | 62 % | 27 % | - |

Der Wirkstoff wird mit den Zusatzstoffen vermischt und in einer geeigneten Mühle homogen vermahlen. Man erhält Spritzpulver, die sich mit Wasser zu Suspensionen jeder gewünschten Konzentration verdünnen lassen.

| 2.2 Emulsions-Konzentrat | |
|---|---|
| Wirkstoff aus den Tabellen | 10 % |
| Octylphenolpolyethylenglykolether (4-5 Mol Ethylenoxid) | 3 % |
| Ca-Dodecylbenzolsulfonat | 3 % |
| Ricinusölpolyglykolether (35 Mol Ethylenoxid) | 4 % |
| Cyclohexanon | 30 % |
| Xylolgemisch | 50 % |

Aus diesem Konzentrat können durch Verdünnen mit Wasser Emulsionen jeder gewünschten Verdünnung hergestellt werden.

| 2.3 Stäubemittel | a) | b) |
|---|---|---|
| Wirkstoff aus den Tabellen | 5 % | 8 % |
| Talkum | 95 % | - |
| Kaolin | - | 92 % |

Man erhält anwendungsfertige Stäubemittel, indem der Wirkstoff mit den Trägerstoffen vermischt und auf einer geeigneten Mühle vermahlen wird.

| 2.4 Extruder Granulat | |
|---|---|
| Wirkstoff aus den Tabellen | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser ange feuchtet. Dieses Gemisch wird extrudiert und anschliessend im Luftstrom getrocknet.

| 2.5 Umhüllungs-Granulat | |
|---|---|
| Wirkstoff aus den Tabellen | 3 % |
| Polyethylenglykol (MG 200) | 3 % |
| Kaolin (MG = Molekulargewicht) | 94 % |

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

| 2.6 Suspensions-Konzentrat | |
|---|---|
| Wirkstoff aus den Tabellen | 40 % |
| Ethylenglykol | 10 % |
| Nonylphenolpolyethylenglykolether (15 Mol Ethylenoxid) | 6 % |
| Na-Ligninsulfonat | 10 % |
| Carboxymethylcellulose | 1 % |
| 37%ige wässrige Formaldehyd-Lösung | 0,2 % |
| Silikonöl als 75% wässrigen Emulsion | 0,8 % |
| Wasser | 32 % |

Der fein gemahlene Wirkstoff wird mit den Zusatzstoffen innig vermischt. Man erhält ein Suspensions-Konzentrat, aus welchem durch Verdünnen mit Wasser Suspensionen jeder gewünschten Verdünnung hergestellt werden können.

### 3, Biologische Beispiele

### Beispiel 3.1: Wirkung gegen Colletotrichum lagenarium auf Cucumis sativus L.

a) Gurkenpflanzen werden nach 2-wöchiger Anzucht mit einer aus Spritzpulver des Wirkstoffes hergestellten Spritzbrühe besprüht (Konzentration: 200 ppm). Nach 72Stunden werden die Pflanzen mit einer Sporensuspension (1.0 . 10⁵ Sporen/ml) des Pilzes infiziert und für 30 Stunden bei hoher Luftfeuchtigkeit und einer Temperatur von 23°C inkubiert. Die Inkubation wird dann bei normaler Luftfeuchtigkeit und bei 22°C bis 23°C weitergeführt. Die Beurteilung der Schutzwirkung erfolgt aufgrund des Pilzbefalls 7-8 Tage nach der Infektion.
b) Gurkenpflanzen werden nach 2-wöchiger Anzucht mit einer aus Spritzpulver des Wirkstoffes hergestellten Spritzbrühe durch Bodenapplikation behandelt (Konzentration: 20 ppm bezogen auf das Bodenvolumen). Nach 72 Stunden werden die Pflanzen mit einer Sporensuspension (1.5 . 10⁵ Sporen/ml) des Pilzes infiziert und für 30Stunden bei hoher Luftfeuchtigkeit und einer Temperatur von 23°C inkubiert. Die Inkubation wird dann bei normaler Luftfeuchtigkeit und bei 22°C weitergeführt.
   Die Beurteilung der Schutzwirkung erfolgt aufgrund des Pilzbefalls 7-8 Tage nach der Infektion.
   Verbindungen aus den Tabellen zeigen in den Tests (a) und (b) gute Wirkung und reduzieren den Pilzbefall auf 0 bis 20 %. Unbehandelte jedoch infizierte Kontrollpflanzen weisen dagegen einen Colletotrichum-Befall von 90 % auf.
c) Vergleichstest: direkte Wirkung gegen Colletotrichum lagenarium.
   Der formulierte Wirkstoff wird in verschiedenen Konzentrationen (100,10,1, 0.1 ppm) mit autoklaviertem und abgekühltem Nährboden, der 10'000 Sporen pro ml enthält, gemischt und in Mikrotiterplatten gegossen. Die Inkubation erfolgt anschliessend bei 22°C im Dunkeln. Nach 2-3 Tagen wird das Pilzwachstum spektrophotometrisch gemessen.
   Bei Verbindungen aus den Tabellen wird keine Hemmung des Pilzwachstum beobachtet; dagegen tritt bei Anwendung des Fungizides "Benomyl" (Handelsprodukt) als Vergleichssubstanz bei 0.2 ppm 50% Hemmung (EC50) des Pilzwachstums auf.

### Beispiel 3,2: Wirkung gegen Phytophthora infestans auf Tomatenpflanzen

a) Tomatenpflanzen werden nach 3-wöchiger Anzucht mit einer aus Spritzpulver des Wirkstoffes hergestellten Spritzbrühe (0,02 % Aktivsubstanz) besprüht. Nach 72 Stunden werden die behandelten Pflanzen mit einer Sporangiensuspension des Pilzes infiziert. Die Beurteilung des Pilzbefalls erfolgt nach einer Inkubation der infizierten Pflanzen während 5 Tagen bei 90-100 % relativer Luftfeuchtigkeit und 20°C.
Verbindungen aus den Tabellen zeigen in den Tests gute Wirkung und reduzieren den Pilzbefall auf 0 bis 20 %. Unbehandelte jedoch infizierte Kontrollpflanzen weisen dagegen einen 60 %igen Phytophthora-Befall auf.

### Beispiel 3,3: Wirkung gegen Pyricularia oryzae auf Reisoflanzen

Zu 2-wöchigen Reispflanzen wird eine aus Spritzpulver des Wirkstoffes hergestellte Spritzbrühe gegossen (0,006 % Aktivsubstanz bezogen auf das Erdvolumen). Darauf werden die Töpfe mit Wasser soweit gefüllt, dass die untersten Stengelteile der Reispflanzen im Wasser stehen. Nach 96 Stunden werden die behandelten Reispflanzen mit einer Konidiensuspension des Pilzes infiziert. Nach einer Inkubation der infizierten Pflanzen während 5 Tagen bei 95 - 100 % relativer Luftfeuchtigkeit und ca. 24°C wird der Pilzbefall beurteilt.
Reispflanzen, die mit einer Spritzbrühe behandelt werden, die als Aktivsubstanz eine Verbindung aus den Tabellen enthält, zeigen im Vergleich zu unbehandelten Kontrollpflanzen (100 % Befall) nur etwa 50% Pilzbefall.

### Beispiel 3,4: Wirkung gegen Cercospora nicotina an Tabakpflanzen

### a) Blattapplikation

Tabakpflanzen (8 Wochen alt) werden mit einer formulierten Lösung des Wirkstoffes besprüht (Konzentration: 0,02 % Aktivsubstanz). Vier Tage nach der Behandlung werden die Pflanzen mit einer Sporangiensuspension von Cercospora nicotina(150 000 Sporen/ml) inokuliert, 5 Tage im Dunkeln bei 25°C und hoher Luftfeuchtigkeit aufbewahrt und dann bei normaler Tag/Nacht Wechselfolge weiterinkubiert.
Die Beurteilung der Symptome in den Tests erfolgt aufgrund der mit Pilz befallenen Blattoberfläche.
Die Kontrollpflanzen zeigen einen Befall von ca. 60 %; Pflanzen, welche mit Verbindungen aus den Tabellen behandelt wurden, zeigen einen Befall von 0-30 %.

### Beispiel 3,5: Wirkung gegen Erysiphe graminis auf Weizen

Protektive Wirkung: 18 Tage alte Weizenpflanzen werden mit einer formulierten Lösung des Wirkstoffes (0,02% Aktivsubstanz) besprüht. Unmittelbar nach der Behandlung werden die Pflanzen unter Zylindem inkubiert. Nach 24 Stunden werden die Pflanzen abgedeckt. Nach weiteren 3 Tagen werden die behandelten Pflanzen oberhalb vom Primärblatt abgeschnitten. Die Primärblätter werden horizontal orientiert und in einer Bestäubungsglocke mit Erysiphe graminis-Sporen inokuliert (Sporendichte:0,2mg/m²). Der Test wird in einer Klimakammer bei 12 h Licht (18KLux), 20°C und 12h Dunkelheit, 18°C durchgeführt. Die Beurteilung des Befalls erfolgt 9 und 13Tage nach Inokulation.
Verbindungen aus den Tabellen zeigen in den Tests gute Wirkung und reduzieren den Pilzbefall auf 0 bis 20 %. Unbehandelte jedoch infizierte Kontrollpflanzen weisen dagegen einen 70%igen Erysiphe-Befall auf.

## Patentansprüche

1. Eine Verbindung der Formel I 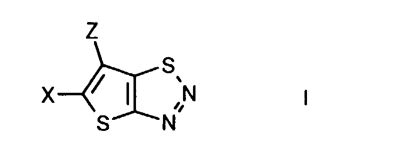 worin bedeuten:
X Wasserstoff, Halogen, C₁-C₄-Alkyl, SR₈, N(R₉)R₁₀, CH₂OH, CHO oder COOR₈; R₈ bis R₁₀ Wasserstoff, C₁-C₄-Alkyl, Benzyl, C₁-C₄-Acyl oder Benzoyl;
Z Wasserstoff, Halogen oder eine Methyl-Gruppe, die unsubstituiert ist oder an die 1-3 gegebenenfalls substituierte Heteroatome O, S und/oder N gebunden sind;
in freier Form oder in Salzform;
mit Ausnahme der Verbindung, worin Z Methyl und X COOC₂H₅ ist.

2. Eine Verbindung der Formel I gemäss Anspruch 1, worin bedeuten:
Z Wasserstoff, CN, CO-A, CS-A, CH(OR₁)_{2,}
und worin ferner die übrigen Substituenten folgende Bedeutungen haben:
A Wasserstoff, Halogen, OR₂, SR₂, N(R₃)R₄, NH-OR₅, O-N(=C)ₙ(R₆)R₇ oder NH-N(=C)ₙ(R₆)R₇;
R₁ gleich oder verschieden C₁-C₄-Alkyl, das unsubstituiert oder durch Phenyl, C₁-C₂ Alkoxy, Phenoxy oder Benzyloxy substituiert ist; oder
zwei OR₁ zusammen mit dem Kohlenstoffatom, an das sie gebunden sind, eine cyclische Acetalgruppe, die unsubstituiert oder mit C₁-C₃-Alkyl, Phenyl, Benzyl, Hydroxy, oder C₁-C₃-Hydroxyalkyl substituiert ist;
R₂ bis R₇ Wasserstoff, ein gegebenenfalls substituierter, bis zu 8 Kohlenstoffatome enthaltender offenkettiger, gesättigter oder ungesättigter Kohlenwasserstoffrest, ein gegebenenfalls substituierter, bis zu 10 Kohlenstoffatome enthaltender cyclischer, gesättigter oder ungesättigter Kohlenwasserstoffrest, gegebenenfalls substituiertes Benzyl oder Phenethyl, eine gegebenenfalls substituierte, bis zu 8 Kohlenstoffatome enthaltende Acylgruppe, eine gegebenenfalls substituierte Benzoylgruppe oder ein gegebenenfalls substituierter Heterocyclylrest; oder
R₃ und R₄ zusammen mit dem Stickstoffatom, an das sie gebunden sind, ein 5- oder 6-gliedriger, gegebenenfalls substituierter Heterocyclus mit 1-3 Heteroatomen O, S, und/oder N;
R₆ und R₇ zusammen mit dem Atom, an welches sie gebunden sind, ein 5- bis 7-gliedriger, gegebenenfalls substituierter carbocyclischer oder heterocyclischer Ring mit 1-3 Heteroatomen O, S, und/oder N;
n 0 oder 1.

3. Eine Verbindung gemäss Anspruch 2, worin bedeuten:
R₁ C₁-C₄-Alkyl, oder zwei OR₁ zusammen mit dem Kohlenstoffatom, an das sie gebunden sind, eine 5-7 gliedrige, cyclische Acetalgruppe, die unsubstituiert oder mit C₁-C₃-Alkyl substituiert ist;
R₂ und R₃ Wasserstoff, C₁-C₈-Alkyl, das unsubstituiert oder mit 1-5 Halogenatomen, C₃-C₆-Cycloalkyl, C₁-C₄-Alkoxy, Phenoxy, Benzyloxy C₁-C₄-Acyloxy, Benzoyloxy, Hydroxy, Nitro, Cyano, C₁-C₄-Alkanoyl, Benzoyl, Carboxyl, C₁-C₄-Alkoxycarbonyl, Benzyloxycarbonyl, Amino, C₁-C₄-Alkylamino, C₁-C₄-Dialkylamino oder Heterocyclyl substituiert ist, C₃-C₆-Alkenyl, das unsubstituiert oder mit 1-5 Halogenatomen substituiert ist, C₃-C₆-Alkinyl, C₃-C₆-Cycloalkyl, C₁-C₄-Alkanoyl, Phenyl, Benzyl oder Phenethyl, deren Phenylringe unsubstituiert oder ein bis dreifach durch Halogen, Hydroxy, C₁-C₄-Alkyl, Haogen-C₁-C₂-alkyl, C₁-C₂-Alkoxy, Halogen-C₁-C₂-alkoxy oder Nitro substituiert sind, Naphthyl, Benzoyl, oder Heterocyclyl, die unsubstituiert oder ein- bis dreifach gleich oder verschieden durch Halogen, C₁-C₂-Alkyl, Halogenmethyl oder Nitro substituiert sind;
R₄ Wasserstoff, C₁-C₆-Alkyl, Benzyl; oder
R₃ und R₄ zusammen mit dem Stickstoffatom, an welches sie gebunden sind, ein 5- oder 6-gliedriger Ring mit 1-2 Heteroatomen O, S, und/oder N,, wobei die genannten Ringe unsubstituiert oder ein- bis zweifach gleich oder verschieden durch Halogen, C₁-C₃-Alkyl oder C₁-C₂-Alkoxycarbonyl substituiert sind;
R₅, R₆, R₇, unabhängig voneinander Wasserstoff, C₁-C₆-Alkyl, C₃-C₄-Alkenyl, C₃-C₄-Alkinyl, C₃-C₆-Cycloalkyl, Phenyl oder Benzyl, deren Phenylringe unsubstituiert oder ein bis zweifach durch Halogen, Hydroxy, C₁-C₂-Alkyl, C₁-C₂-Alkoxy, Halogenmethyl oder Halogenmethoxy substituiert sind; oder
R₆ und R₇ zusammen mit dem Atom, an welches sie gebunden sind, ein 5- bis 7-gliedriger carbocyclischer oder heterocyclischer Ring mit 1-3 Heteroatomen O, S und/oder N, wobei die genannten Ringe unsubstituiert oder ein- bis dreifach gleich oder verschieden durch Halogen oder C₁-C₃-Alkyl substituiert sind.

4. Eine Verbindung der Formel I gemäss Anspruch 1, worin bedeuten:
Z CO-A, CS-A, CH(OR₁)_{2,}

5. Eine Verbindung der Formel I gemäss Anspruch 1, worin bedeuten:
Z CO-A;
A Wasserstoff, OR₂, SR₂, N(R₃)R₄, NH-OR₅, O-N(=C)ₙ(R₆)R₇ oder NH-N(=C)ₙ(R₆)R₇;
R₂ und R₃ Wasserstoff, C₁-C₈-Alkyl, das unsubstituiert oder mit 1-5 Halogenatomen, C₃-C₆-Cycloalkyl, C₁-C₄-Alkoxy, Phenoxy, Benzyloxy, C₁-C₄-Acyloxy, Benzoyloxy, Hydroxy, Nitro, Cyano, C₁-C₄-Alkanoyl, Benzoyl, Carboxyl, C₁-C₄-Alkoxycarbonyl, Benzyloxycarbonyl, Amino, C₁-C₄-Alkylamino, C₁-C₄-Dialkylamino oder Heterocyclyl substituiert ist, C₃-C₆-Alkenyl, das unsubstituiert oder mit 1-5 Halogenatomen substituiert ist, C₃-C₆-Alkinyl, C₃-C₆-Cycloalkyl, C₁-C₄-Alkanoyl, Phenyl, Benzyl oder Phenethyl, deren Phenylringe unsubstituiert oder ein bis dreifach durch Halogen, Hydroxy, C₁-C₄-Alkyl, Halogen-C₁-C₂-alkyl, C₁-C₂-Alkoxy, Halogen-C₁-C₂-alkoxy oder Nitro substituiert sind, Naphthyl, Benzoyl, oder Heterocyclyl, die unsubstituiert oder ein- bis dreifach gleich oder verschieden durch Halogen, C₁-C₂-Alkyl, Halogenmethyl oder Nitro substituiert sind, oder
R₃ und R₄ zusammen mit dem Stickstoffatom, an welches sie gebunden sind, ein 5- oder 6-gliedriger Ring mit 1-2 Heteroatomen O, S, und/oder N, wobei die genannten Ringe unsubstituiert oder ein- bis zweifach gleich oder verschieden durch Halogen, oder C₁-C₃-Alkyl substituiert sind;
R₅, R₆, R₇, unabhängig voneinander Wasserstoff, C₁-C₆-Alkyl, C₃-C₄-Alkenyl, C₃-C₄-Alkinyl, C₃-C₆-Cycloalkyl, Phenyl oder Benzyl, deren Phenylringe unsubstituiert oder ein bis zweifach durch Halogen, Hydroxy, C₁-C₂-Alkyl, C₁-C₂-Alkoxy, Halogenmethyl oder Halogenmethoxy substituiert sind; oder
R₆ und R₇ zusammen mit dem Atom, an welches sie gebunden sind, ein 5- bis 7-gliedriger carbocyclischer oder heterocyclischer Ring mit 1-3 Heteroatomen O, S und /oder N, wobei die genannten Ringe unsubstituiert oder ein- bis dreifach gleich oder verschieden durch Halogen, C₁-C₃-Alkyl oder C₁-C₂-Alkoxycarbonyl substituiert sind.

6. Eine Verbindung der Formel I gemäss Anspruch 5, worin bedeuten:
Z CO-A;
A OR₂ oder SR₂.

7. Eine Verbindung der Formel I gemäss Anspruch 6, worin bedeuten:
R₂ Wasserstoff, C₁-C₆-Alkyl, das unsubstituiert oder mit 1-3 Halogenatomen, C₃-C₆-Cycloalkyl, C₁-C₂-Alkoxy, Phenoxy, Hydroxy, Cyano, C₁-C₂-Alkanoyl, Benzoyl, Carboxyl, C₁-C₄-Alkoxycarbonyl, Benzyloxycarbonyl, C₁-C₂-Acyloxy, Benzoyloxy, Amino, C₁-C₄-Alkylamino oder C₁-C₄-Dialkylamino substituiert ist, C₃-C₄-Alkenyl, das unsubstituiert oder mit 1-3 Halogenatomen substituiert ist, C₃-C₄-Alkinyl, C₃-C₆-Cycloalkyl, Phenyl, Benzyl oder Phenethyl, deren Phenylringe unsubstituiert oder ein bis zweifach durch Halogen, Hydroxy, C₁-C₄-Alkyl, Halogen-C₁-C₂-alkyl, C₁-C₂-Alkoxy, Halogen-C₁-C₂-alkoxy oder Nitro substituiert sind;

8. Eine Verbindung der Formel I gemäss Anspruch 2, worin
Z CO-OR₂ bedeutet.

9. Eine Verbindung der Formel I gemäss Anspruch 1, worin bedeuten:
Z CN oder CO-N(R₃)R₄ ;
R₃ Wasserstoff, C₁-C₅-Alkyl, das unsubstituiert oder mit 1-3 Halogenatomen, C₃-C₆-Cycloalkyl, C₁-C₂-Alkoxy, Phenoxy, Hydroxy, Cyano, C₁-C₂-Alkanoyl, Benzoyl, Carboxyl, C₁-C₄-Alkoxycarbonyl, Benzyloxycarbonyl, C₁-C₂-Acyloxy, Benzoyloxy, Amino, C₁-C₄-Alkylamino oder C₁-C₄-Dialkylamino substituiert ist, C₃-C₄-Alkenyl, das unsubstituiert oder mit 1-3 Halogenatomen substituiert ist, C₃-C₄-Alkinyl, C₃-C₆-Cycloalkyl, Phenyl, Benzyl oder Phenethyl, deren Phenylringe unsubstituiert oder ein bis zweifach durch Halogen, Hydroxy, C₁-C₄-Alkyl, Halogen-C₁-C₂-alkyl, C₁-C₂-Alkoxy, Halogen-C₁-C₂-alkoxy oder Nitro substituiert sind;
R₄ Wasserstoff, C₁-C₆-Alkyl, Benzyl; oder
R₃ und R₄ zusammen mit dem Stickstoffatom, an das sie gebunden sind, Pyrrolidin, Piperidin, Morpholin oder Dimethylmorpholin.

10. Eine Verbindung der Formel I gemäss Anspruch 2, worin bedeuten
Z CHO oder CH(OR₁)₂;
R₁ gleich oder verschieden C₁-C₄-Alkyl, das unsubstituiert oder durch Phenyl, C₁-C₂ Alkoxy, Phenoxy oder Benzyloxy substituiert sein kann; oder zwei OR₁ zusammen mit dem Kohlenstoffatom, an das sie gebunden sind eine 5 oder 6-gliedrige, cyclische Acetalgruppe, die unsubstituiert oder mit C₁-C₃-Alkyl, Phenyl, Benzyl, Hydroxy, oder C₁-C₃-Hydroxyalkyl substituiert ist.

11. Eine Verbindung der Formel I gemäss Anspruch 1, worin
Z Wasserstoff, Methyl, Halogen, C₁-C₄-Alkoxymethyl oder Halogenmethyl bedeuten.

12. Eine Verbindung der Formel I gemäss Anspruch 1, worin
X Wasserstoff, Halogen, Methyl, Mercapto, Methylthio oder Amino bedeutet.

13. Eine Verbindung der Formel 1 gemäss Anspruch 1, worin
X Wasserstoff bedeutet.

14. Verfahren zur Herstellung einer Verbindung der Formel la, 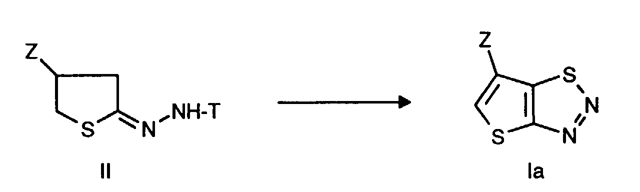 worin Z die für Formel I angegebenen Bedeutungen hat, dadurch gekennzeichnet, dass man eine Verbindung der Formel II, worin T eine Gruppe CO-Y, CONY₂ oder SO₂-Y und Y Wasserstoff, gegebenenfalls substituiertes C₁-C₈₋-Alkyl oder gegebenenfalls substituiertes Aryl bedeuten oder T eine Gruppe COO-U und U ein gegebenenfalls substituiertes C₁-C₈₋-Alkyl oder gegebenenfalls substituiertes Aryl bedeutent, mit Thionylchlorid umsetzt.

15. Verfahren zur Herstellung einer Verbindung der Formel I, 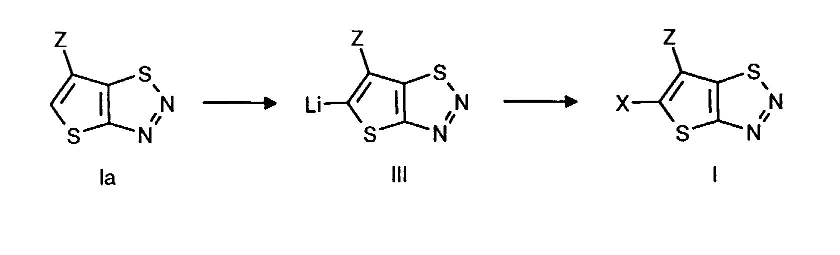 worin X und Z die für Formel I angegebenen Bedeutungen haben, dadurch gekennzeichnet, dass man eine Verbindung der Formel la, worin Z die für Formel I angegebenen Bedeutungen hat, mit einer lithiumorganischen Verbindung wie z.B. Butyllithium, sec.Butyllithium, Hexyllithium, Lithiumdiisopropylamid (LDA), Lithiumhexamethyldisilazid oder Lithiumtetramethylpiperidid (LTMP) in einem aprotischen Lösungsmittel wie z.B. THF, Diethylether oder Hexan in An- oder Abwesenheit eines zusätzlichen Komplexierungsmittels bei -100°C bis +20°C zu einer lithiumorganischen Verbindung der Formel III umsetzt und diese weiterumsetzt
a) zur Herstellung einer Verbindung, worin X Halogen bedeutet, mit einem Halogen;
b) zur Herstellung einer Verbindung, worin X Chlor bedeutet, mit Hexachlorethan;
c) zur Herstellung einer Verbindung, worin X C₁-C₄-Alkyl bedeutet, mit einemC₁-C₄-Alkylhalogenid;
d) zur Herstellung einer Verbindung, worin X CH₂OH bedeutet, mit Formaldehyd;
e) zur Herstellung einer Verbindung, worin X CHO bedeutet, mit Dimethylformamid.;
f) zur Herstellung einer Verbindung, worin X COOR₈ bedeutet, mit CO₂ und gegebenenfalls anschliessende verestert.

16. Verfahren zur Herstellung einer lithiumorganischen Verbindung der Formel III 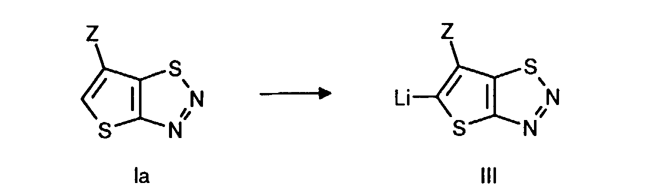 worin Z die für Formel I angegebenen Bedeutungen hat, dadurch gekennzeichnet, dass man eine Verbindung der Formel la, worin Z die für Formel I angegebenen Bedeutungen hat, mit einer lithiumorganischen Verbindung wie z.B.Butyllithium, sec.Butyllithium, Hexyllithium, Lithiumdiisopropylamid (LDA), Lithiumhexamethyldisilazid oder Lithiumtetramethylpiperidid (LTMP) in einem aprotischen Lösungsmittel wie z.B. THF, Diethylether oder Hexan in An- oder Abwesenheit eines zusätzlichen Komplexierungsmittels bei -100°C bis +20°C umsetzt.

17. Verfahren zur Herstellung einer Verbindung der Formel I 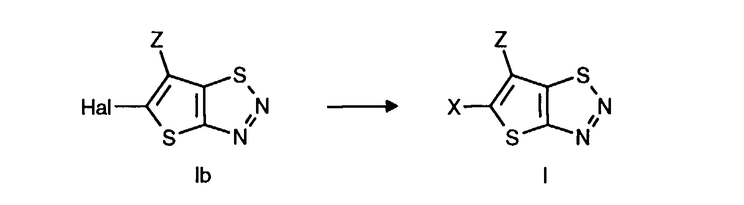 worin Z die für Formel I angegebenen Bedeutungen hat und X SR₈ oder N(R₉)R₁₀ bedeuten, dadurch gekennzeichnet, dass man eine Verbindung der Formel Ib, worin Hal Halogen bedeutet, mit HSR₈ oder HN(R₉)R₁₀ unter basischen Bedingungen umsetzt.

18. Eine Verbindung der Formel 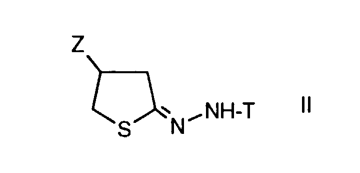 worin Z die für Formel I angegebenen Bedeutungen hat und T eine Gruppe CO-Y, COO-Y, CONY₂ oder SO₂-Y und Y Wasserstoff, gegebenenfalls substituiertes C₁-C₈₋-Alkyl oder gegebenenfalls substituiertes Aryl bedeuten.

19. Eine lithiumorganische Verbindung der Formel III 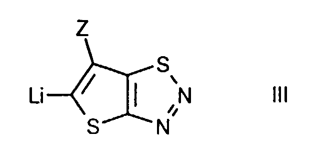 worin Z die für Formel I angegebenen Bedeutungen hat, als Lösung in einem aprotischen Lösungsmittel.

20. Mittel zum Schutz von Pflanzen gegen den Befall durch Mikroorganismen, dadurch gekennzeichnet, dass es als Wirkstoff eine Verbindung gemäss Anspruch 1, in freier Form oder in agrochemisch verwendbarer Salzform, zusammen mit einem Trägermaterial enthält.

21. Verfahren zum Schutz von Pflanzen gegen den Befall durch Mikroorganismen, dadurch gekennzeichnet, dass man als Wirkstoff eine Verbindung der Formel I gemäss Anspruch 1 auf die Pflanzen, auf Teile der Pflanzen und/oder auf den Standort der Pflanzen appliziert.

22. Verfahren zur Immunisierung von Pflanzen gegen den Befall durch Mikroorganismen, dadurch gekennzeichnet, dass man als Wirkstoff eine Verbindung der Formel I gemäss Anspruch 1 auf die Pflanzen, auf Teile der Pflanzen und/oder auf den Standort der Pflanzen appliziert.
